# EUROPEAN PATENT APPLICATION

(11) **EP 4 512 348 A1**
(43) Date of publication of application: **26.02.2025**
(21) Application number: 23315327.9
(22) Date of filing: 25.08.2023
(51) Int. Cl.: A61B 17/04, A61F 5/00, A61B 17/068, A61B 17/12, A61B 17/02, A61B 17/00, A61B 17/30

(54) **A MEDICAL APPARATUS FOR CARRYING OUT AN ENDOSCOPIC GASTRIC REMODELLING PROCEDURE**

(71) Applicant: Caranx Medical, 75013 Paris (FR)
(72) Inventor: BERTHET-RAYNE, 06800 Cagnes-sur-Mer (FR); CERRUTI, Giulio, 06700 Saint-Laurent-Du-Var (FR); SEJOR, Eric, 06000 Nice (FR); HARMEN SMITS, Jonas Victor, 3360 Bierbeek (BE); SCHEGG, Pierre, 06000 Nice (FR); LITTLE, Kieran, 06000 Nice (FR); POULETTY, Philippe, 75005 Paris (FR)
(74) Representative: Hepp Wenger Ryffel AG

(57) **Abstract**

The invention relates to a medical apparatus (101) for carrying out an endoscopic gastric remodelling procedure comprising a sizing tube (2) which has an inner lumen (21), at least one vacuum port (22) for applying suction to stomach tissue (19) and forming at least two tissue bulges (191, 192) of the stomach tissue (19), and at least one tissue receiving opening (23, 24) extending in a longitudinal direction (L) of the sizing tube (2) for receiving the tissue bulges (191, 192) based on the applied suction. The medical apparatus (101) has a puncturing connection unit (3), characterized in that the puncturing connection unit (3) is adapted for connecting the two tissue bulges (191, 192) with each other by puncturing the tissue bulges (191, 192), and an expansion unit (11) for spreading the stomach which has a collapsed configuration (111) and an expanded configuration (112), wherein the expansion unit (11) in the expanded configuration is configured for engaging the greater curvature (196)of the stomach and advancing the sizing tube towards the lesser curvature (195) of the stomach.

## Description

The present invention relates to a medical apparatus and a method for carrying out an endoscopic gastric remodelling procedure according to the independent claims.

Obesity is associated with a wide variety of diseases and comorbidities posing significant health risks and may result in severe medical conditions such as Type 2 diabetes, high blood pressure, apnea, dyslipidemia and certain types of cancer.

Minimally invasive bariatric surgery, in particular by gastric volume restriction/reduction, has been shown to provide a most effective way for achieving improved long-term sustained weightloss outcomes while minimizing surgical complications.

Nonetheless, bariatric surgery may be subject of health risks such as bleeding and/or leakage into the abdominal cavity which may in severe cases lead to infections, e.g. peritonitis, or even death. Therefore, it is of paramount importance to enable precise and efficient connection of stomach tissue which reduces surgical complications, provides post-surgery infection risk mitigation, and in particular may be tailored to the specific anatomy/needs of a patient.

US 2022/079577 A1 discloses a bougie capsule section which is configured for shaping gastric tissue and suturing the tissue via an arcuate needle which may be driven by a rotatable drive by friction between a shaft and the needle.

EP 4 005 503 A1 discloses a needle assembly with a suturing device which is adapted for placing tubular needle bodies with a suture extending through the needle bodies and the needle bodies are adapted to be longitudinally displaceable along a length of the suture.

The prior art lacks a medical apparatus which allows to connect stomach tissue in a simple and reliable manner such that tissue bulges are connected at spatially optimized locations and have ideal dimensions.

Moreover, the prior art lacks an optimized positioning of the optimized locations which is not dependent on the skills of a clinician. In particular, the prior art fails to disclose a medical apparatus which is configured for providing a more uniform and predictable suturing surface of the tissue bulges of the gastric tissue.

It is the objective of the present invention to overcome these and other disadvantages of the prior art and provide a medical apparatus and method for carrying out an endoscopic gastric remodelling procedure. The medical apparatus should further facilitate the surgical procedure, minimize surgical complications, and postoperative morbidity. These objectives are solved by the medical apparatus and method according to the independent claims. Further embodiments result from the dependent claims.

The medical apparatus for carrying out an endoscopic gastric remodelling procedure comprises a sizing tube which has an inner lumen, at least one vacuum port for applying suction to stomach tissue and forming at least two tissue bulges of the stomach tissue. The sizing tube has at least one tissue receiving opening extending in a longitudinal direction of the sizing tube for receiving the tissue bulges based on the applied suction. The medical apparatus further comprises a puncturing connection unit, which is adapted for connecting the at least two tissue bulges with each other by puncturing the tissue bulges. The medical apparatus preferably comprises an expansion unit for spreading the stomach. The expansion unit has a collapsed configuration and an expanded configuration. The expansion unit in the expanded configuration is configured for engaging the greater curvature of the stomach and for displacing the greater curvature away from the sizing tube.

The puncturing connection unit may be configured to connect the tissue bulges by deploying fastening devices, such as fasteners, barbs, clips and/or sutures. The puncturing connection unit may also be adapted to deploy biocompatible adhesives for enhancing the connection of the tissue bulges.

In comparison with minimally invasive surgical procedures (sleeve gastroplasty, Roux-en-Y gastric bypass, single-anastomosis gastric bypass, gastric banding), endoscopic gastric remodelling is a less invasive surgical procedure, in which the stomach size / effective volume is reduced by connecting, e.g. ventral and dorsal, parts of the stomach to create a tubular sleeve formed by the native tissue of a patient. This enables to segment the stomach and precisely adjust a portion of the stomach to receive incoming food. The medical apparatus may be adapted for carrying out an endoscopic peroral endosleeve surgery.

The expansion unit allows to flatten the stomach tissue and increase a tissue spread of the stomach such that unintended folding of stomach tissue, in particular transverse to the tube, can be prevented. These tissue folds may otherwise result in uneven/non-uniform connection of tissue bulges.

In addition, the expansion unit may enable a reliable placement of the sizing tube along the lesser curvature of the stomach by pushing the greater curvature of the stomach away from the sizing tube.

The expansion unit may comprise an inflatable structure which has a collapsed configuration and an expanded configuration. In the expanded configuration, the inflatable structure is adapted for displacing the greater curvature away from the sizing tube.

The expansion unit may be designed to allow the receiving opening(s) to be at least partially unobstructed, such that the expansion unit in the expanded configuration allows the tissue bulges to be received in the receiving opening(s).

The expansion unit may be connected to a proximal and/or a distal end of the sizing tube. The expansion unit in the collapsed configuration may extend along a longitudinal direction of the sizing tube in a parallel or in an arcuate manner, in particular may longitudinally extend along the entire tissue receiving opening(s).

The expansion unit may comprise an elongated element, in particular a hyperelastic elongated element, which has a collapsed configuration and an expanded configuration. The elongated element in the expanded configuration is adapted for displacing the greater curvature away from the sizing tube, in particular by the elongated element interfacing at least partially tangentially with the greater curvature'. The elongated element may have an arcuate shape with a smaller radius in the expanded configuration than in the collapsed configuration.

The elongated element may be a wire. The expansion unit may comprise or consist of a hyperelastic material, preferably a shape memory material, in particular nitinol.

The expansion unit may be reversibly convertible between the collapsed and expanded configuration by longitudinally advancing/retracting the elongated element, in particular to progressively adapt an arcuate curvature of the expansion unit, in particular of the elongated element. This design of the expansion unit enables a simple and reliable deployment which evenly distributes the pressure along the greater curvature of the stomach to remove gastric folds.

The design of the expansion unit may further be configured to apply lateral tension to the dorsal and ventral stomach wall which facilitates formation of the.tissue bulges and simplifies the endoscopic gastric remodelling of the stomach.

The expansion unit may be configured have an expanded configuration in which the expansion unit laterally extends a distance between 4 cm to 18 cm, in particular a distance between 5 cm to 14 cm, preferably a distance between 7 cm to 10 cm, away from the sizing tube.

The expansion unit may extend a distance between 3 cm to 24 cm, in particular between 5 cm to 20 cm, preferably between 7 cm and 15 cm, along the longitudinal direction of the sizing tube in the collapsed and/or expanded configuration.

The medical apparatus may comprise a control unit which is configured for operating a fluid flow into/out of the stomach, e.g. through the at least one vacuum port. The control unit may be adapted for controlling a fluid inflow into the stomach and/or a fluid outflow from the stomach to facilitate a simultaneous or synchronized deployment of the expansion unit. The control unit may be adapted for providing cyclic inflow and outflow of fluid through the vacuum port in a synchronized manner with moving the expansion unit from the collapsed to the expanded configuration. The control unit may be adapted-for converting the expansion unit completely to the expanded configuration, in particular while continuously increasing an applied fluid inflow, prior to applying suction for forming the tissue bulges.

The puncturing connection unit may longitudinally extend along the at least one longitudinal tissue receiving opening, in particular along the entire at least one tissue receiving opening. The puncturing connection unit may be configured to be translationally immovable along the longitudinal direction, in particular fixedly connected to the sizing tube. The puncturing connection unit may be configured to be rotationally movable, in particular in unison with the sizing tube.

The medical apparatus may comprise an actuator for rotating the puncturing connection unit, in particular together with the sizing tube or relative to the sizing tube.

The medical apparatus may have at least two tissue receiving openings which are connected to the inner lumen of the sizing tube and are at least partially separated by an elongated partitioner of the sizing tube. The two tissue receiving openings may be arranged parallel to each other and/or may have a same longitudinal length. The first tissue receiving opening is adapted for receiving the first tissue bulge and the second tissue receiving opening is adapted for receiving the second tissue bulge when the suction is applied.

The elongated partitioner may further be adapted such that the first tissue opening receives a first tissue bulge of the ventral stomach tissue and the second tissue opening receives a second tissue bulge of the dorsal stomach wall. This allows for reliably performing an endoscopic gastric remodelling procedure by connecting the tissue bulges of the stomach tissue.

This elongated partitioner allows for a reliable separate formation of the tissue bulges which are received in the receiving openings based on the applicable suction and preferably enables formation of uniformly sized tissue bulges.

The elongated partitioner may be adapted such that the at least one tissue receiving opening, in particular both tissue receiving openings, are arrangeable on the lateral side of the sizing tube in the stomach that faces the greater curvature of the stomach rather than the lesser curvature of the stomach. This allows a simpler connection of the ventral and dorsal tissue bulges and optimized shape and sizing of the resulting endoscopic gastric remodelling.

The medical apparatus may have pairs of tissue receiving openings which are spaced apart from each other in a circumferential direction of the sizing tube. A plurality of pairs of tissue receiving openings may be arranged distanced from each other along the longitudinal direction of the sizing tube, in particular in an equidistant manner. This provides predefined puncturing connection positions at which the puncturing connection unit can connect the tissue bulges received by the pairs of tissue receiving openings. The tissue receiving openings may have a rectangular, polygonal, circular or elliptical shape.

The elongated partitioner may be attached on both ends to the sizing tube and immovable with respect to the sizing tube. The puncturing connection unit may be arranged laterally outwardly, with respect to the elongated partitioner. The puncturing connection unit may be adapted to connect the at least two tissue bulges at a location laterally outwardly with respect to the elongated partitioner and the sizing tube. This allows the puncturing connection unit to connect the tissue bulges in a manner that the elongated partitioner is .not enclosed amidst the tissue bulges and deployed sutures/fasteners such that a lateral removal of the elongated partitioner from between the tissue bulges can be achieved.

The elongated partitioner of the sizing tube may have one free end which is not connected to the sizing tube and one joined end which is connected to the sizing tube. Alternatively, the elongated partitioner may have two joined ends which are connected to the sizing tube with at least one of the joined ends being separable from the sizing tube. The elongated partitioner may have a non-flexible state, in particular a non-flexible state having a bent shape, which can be converted into a more flexible state by actuation of a conversion element, in particular by retraction of the conversion element from the elongated partitioner. In the flexible state, a plurality of separated longitudinal subsections of the elongated partitioner are preferably freely movably connected to each other by at least one flexible connecting element, in particular a cable. The elongated partitioner may comprise or be formed by a self-dissolving, soluble and/or bioresorbable material.

Alternatively, the elongated partitioner may be movable with respect to the sizing tube, in particular retractable in a proximal direction with respect to the sizing tube. This provides for a simple removal of the elongated partitioner such that the two tissue receiving openings form a single tissue receiving opening, e.g. after deploying suture / fasteners through the tissue bulges.

This enables a simple.removal of the elongated partitioner from between the connected tissue bulges once the tissue bulges were connected by the puncturing connection unit and provides a space-efficient design of the medical apparatus.

The elongated partitioner may be monolithically formed with the sizing tube. The elongated partitioner may be longitudinally retractable in a proximal direction from an advanced position at least partially separating the two tissue receiving openings to a retracted position in which the two tissue receiving openings are merged to one tissue receiving opening. This allows for a simple ,removal of the elongated partitioner from amidst the connected tissue bulges.

The elongated partitioner and/or the at least one receiving opening may extend over the majority of the sizing tube, in particular more than 80%, in particular more than 90%, of the length of the sizing tube. This allows a reliable formation of tissue bulges along most of a longitudinal length of the sizing tube.

The inner lumen of the sizing tube may have a first terminal opening and a second terminal opening on an opposing side from the first terminal opening, such that the sizing tube may be advanced over a medical instrument, in particular a guidewire, a balloon catheter, an endoscope, and/or an ultrasound device. The medical apparatus may comprise the medical instrument.

The terminal openings enable a simple and facilitated positioning/alignment of the medical instrument within the stomach, e.g. by prior advancing a more intricate medical instrument perorally through the oesophagus into the pylorus.

The medical apparatus may have at least one sealing unit for sealing an outlet from the stomach and/or an inlet into the stomach. The sealing unit may comprise a first sealing element and a second sealing element, preferably formed by a balloon. The first sealing element is adapted for sealing the outlet and the second sealing element is adapted for sealing the inlet.

This allows to seal the inlet and outlet of the stomach, such that localized suction can be applied only to the stomach via the vacuum port. Thus, the required pressure for forming the tissue bulges and the necessary suction to move the tissue bulges into the receiving opening(s) can be minimized. The vacuum port may comprise a perforated barrier which may be adapted to prevent clogging of the vacuum port and/or uniformly distributing a negative pressure of the vacuum port, e.g., along the tissue receiving opening.

The at least one sealing element, in particular the first sealing element for sealing the outlet, may further provide a reference position for the subsequent endoscopic gastric remodelling procedure once deployed to at a sealing position.

Additionally or alternatively, the medical instrument, e.g. a guidewire, catheter, or endoscope, may comprise the sealing unit, in particular the first and second sealing elements. At least one sealing element may be movable along the medical instrument or sizing tube such that the longitudinal position can be adjusted according to the stomach size.

The sizing tube may be at least partially radially expandable, in particular inflatable, preferably along an entire longitudinal length of the sizing tube. The sizing tube may be sized and shaped to be radially expandable from a first cross-sectional dimension of the sizing tube having a value between 1 mm to 20 mm, in particular 2 mm to 16 mm, preferably 4 mm to 12 mm, to a second cross-sectional dimension having a value between 10 mm35 mm, in particular 15 mm - 25 mm, preferably 18 mm - 25 mm.

This enables a facilitated insertion of the sizing tube through the oesophagus and at the same time enables to adjust the cross-sectional dimension of the endoscopic gastric remodelling, such as a diameter of the endosleeve, can be adjusted, e.g. in accordance with the specifics of the surgical intervention, individual characteristics of patients, and/or the indication and clinician's decisions during the intervention.

The sizing tube may be sized and shaped to be uniformly radially expandable, preferably without radially expanding specific sections of the sizing tube to a larger degree.

The sizing tube may further be adapted such that a collapsed configuration is more flexible to facilitate insertion into the stomach and an expanded configuration, in particular radially expanded configuration, is more rigid, and in particular has a bent shape, for facilitating the surgical intervention in the stomach.

The sizing tube may comprise an inflatable section which renders the sizing tube radially expandable, in particular comprising a balloon. A mantle surface of the sizing tube circumferentially around the sizing tube without covering the receiving opening(s) may be expanded, in particular by inflating a flexible membrane of the inflatable section. The flexible membrane of the sizing tube may be adapted to expand in a uniform manner, such that the mantle surface of the sizing tube radially increases to the same extend. Alternatively, the flexible membrane may have an expanded state in which a radial dimension of the mantle surface varies along the length of the sizing tube. A proximal end of the flexible membrane in the expanded state may have a different radial dimension than a distal end. In the expanded state, the mantle surface may define an at least partially conical shape.

This may allow for adapting the stomach remodelling to the specific anatomy / indication of the patient and improve patient outcomes.

A movable puncturing connection unit may at least be partially arranged enclosed and/or neighbouring an inflatable section of the sizing tube. This enables reduced friction; e.g. with stomach tissue, when translationally/rotationally moving the puncturing connection unit, if the inflatable section is inflated to radially expand the sizing tube.

A volume of the sizing tube, or at least the section of the sizing tube adapted for insertion into the stomach may be between 50 ml - 350 ml, in particular 75 ml - 250 ml, preferably 100 ml - 200 ml in a target position.

This allows to perform the endoscopic gastric remodelling procedure on patients to constrain nutrition uptake while enabling safe digestion and assimilation of food.

If the sizing tube is radially expandable, the values of the volume of the sizing tube relates to the sizing tube in the previously described radially expanded state.

The puncturing connection unit may be configured to translationally move in the longitudinal direction with respect to the sizing tube. This allows a more customizable connection of the tissue bulges tailored to the indication of the patient.

The puncturing connection unit may comprise at least one puncturing element, an actuation unit for driving the puncturing element, and in particular a translation unit for translationally moving the actuation unit in the longitudinal direction/rotational direction. The translation unit may be adapted to interact with a guiding feature for translationally/rotationally moving the puncturing connection unit.

The translational/rotational movement of the puncturing connection unit allows for an optimized connection of the tissue bulges according to the anatomy of the patient, in particular in a sequential manner. The actuation unit may be configured for piercing the tissue bulges at pre-defined or user-defined positions which increases the reliability/versatility of the apparatus.

The puncturing connection unit may comprise a plurality of puncturing elements which may be driven by the at least one actuation unit, in particular in a synchronized manner, preferably in unison. The puncturing elements may be spaced apart from each other in the longitudinal direction of the sizing tube. The puncturing elements may be circumferentially arranged with respect to the sizing tube and in particular advanceable along a circumferential direction of the sizing tube, optionally without being movable in the longitudinal direction.

The puncturing connection unit may be arranged (i) partially or completely within an inner lumen of the sizing tube, in particular partially within the at least one tissue receiving opening, (ii) around a surgical instrument within the sizing tube, (iii) around the sizing tube and/or (iv) around an elongated partitioner of the sizing tube formed between to openings, in particular formed between two tissue receiving openings, of the sizing tube.

Arranging the puncturing connection unit according to (i) or (ii) allows for a more compact design of the medical apparatus.

The puncturing connection unit may be longitudinally movably arranged or arrangeable with respect to the sizing tube, in particular slidable along a guiding feature of the sizing tube.

This allows for a simple translational movability of the puncturing connection unit to connect the tissue bulges while suction pressure is applied.

The puncturing connection unit which is arranged within the inner lumen of the sizing tube may be slidable along a guiding feature in the inner lumen, in particular a guiding feature extending in a longitudinal or helical direction along the inner lumen. A helical movement of the puncturing connection unit, in particular of the puncturing element, e.g. a helical needle, may allow for connecting, in particular suturing or deploying fasteners, at predetermined, preferably equidistant, longitudinal distances of the sizing tube through the tissue bulges. The guiding feature may be formed by a helical groove extending along the inner lumen for guiding the helical puncturing element.

The puncturing connection unit may be actuatable along the guiding track via a push/pull rod which is configured to be moved longitudinally and/or rotationally.

The medical apparatus, in particular a proximal end of the puncturing connection unit, may comprise a moveable sealing structure which can be moved / changed for partially sealing the receiving opening(s). Thereby, a location and length of the at least one vacuum port may be changed along the longitudinal direction of the sizing tube. The sealing structure may be configured for spatially confining the at least one vacuum port to a customizable subsection of the receiving opening(s). Additionally or alternatively, a spatial position of a proximal end of the vacuum port may be defined by the sealing structure independently from a distal end of the vacuum port. This allows a vacuum to be applied in a more targeted manner so that a larger suction can be applied at a specific confined location, e.g. only at a specific connection region for connecting the two tissue bulges.

Arranging the puncturing connection unit according to.(iii) or (iv) is particularly desirable, in particular for a design having two-receiving openings separated by the elongated partitioner, because the puncturing connection unit can be arranged at a radial more outwards position with respect to the sizing tube. This enables the puncturing connection unit to engage an ideal shape and size of tissue bulges at the radially outward position for an optimized connection, in particular with a minimum application of suction pressure.

The puncturing connection unit may be arranged such that the puncturing connection unit at least partially circumferentially encloses the sizing tube and/or the elongated partitioner. The apparatus may comprise a plurality of nested tubular elements which are.movable with respect to each other without changing the total longitudinal dimension of the tubular elements. The nested tubular elements may extend along the longitudinal direction of the sizing tube, and in particular circumferentially enclose the sizing tube at least partially. The puncturing connection unit may be attached to on one of the tubular elements such that resistive force when moving the puncturing connection unit along the receiving opening(s) is reduced.

The sizing tube may have a bent shape or may be deflectable to a bent shape. The bent shape may have an angle between 10° - 110°, in particular between 20° - 100°, over an entire or partial longitudinal extension of the sizing tube. The longitudinal extension of the sizing tube may be shaped or deflectable corresponding to the gastric course along the lesser curvature of the stomach.

The bent shape allows the sizing tube to be deflected/deflectable to conform to the specific anatomy of the patient. The sizing tube may be deflected by actuating means of the medical apparatus such as at least one pull wire or push rod. Alternatively, the actuating means may be formed by at least one additional vacuum port which can be aligned with the lesser curvature of the stomach wall such that an applied suction enables the sizing tube to be deflected and/or that the lesser curvature of the stomach wall is suctioned and positionable.adjacent to the sizing tube.

The puncturing connection unit may have at least one puncturing element, in particular a curved puncturing element, which is functionally connected or connectable to an implantable connecting structure, in particular to a fastener or a suture. The puncturing connection unit may be adapted for deploying the connecting structure through the tissue bulges by advancing the puncturing element with the connecting structure through the tissue bulges and preferably retracting the puncturing element.

This allows an efficient connection between the tissue bulges. In particular, a curved puncturing element provides a curved penetration path and reduces the tension of the connecting structure once it was deployed and allows for a compact design, in particular if the at least one curved puncturing element is essentially arranged circumferentially or helically to the sizing tube. The puncturing connection unit may be adapted to advance the curved puncturing element in the circumferential or helical direction of the sizing tube.

The puncturing connection unit may comprise at least a first puncturing element having a curved shape with a first diameter and a second puncturing element having a curved shape with a second diameter. The first diameter is different from the second diameter.

Alternatively or additionally, the puncturing connection unit may have one puncturing element which has two curved puncturing regions that have two different diameters.

The puncturing connection unit may comprise a plurality of puncturing elements having the same diameter which are arranged for puncturing the tissue in a zig-zag pattern.

This may prevent leakage to a greater extend by enabling punctures through the tissue bulges at different heights and thus dimensions of the tissue bulges.

This allows the puncturing connection unit to connect the tissue bulges at two laterally spaced apart puncturing sites via the puncturing element(s) with respect to the sizing tube, e.g. by placing laterally separated sutures / fasteners.

Alternatively, the puncturing element may be arranged parallel to the longitudinal direction of the sizing tube and translationally movable along the longitudinal direction, if a plurality of bulges is arrangeable along the longitudinal direction of the sizing tube.

The puncturing element may be adapted to release the connecting structure once it was deployed. The connecting structure may be bioresorbable, self-dissolving, or soluble. The connecting structure may comprise anchors, such as barbed anchors or helical anchors and in particular a suture.

An effective length of the suture of the connecting structure can be increased or decreased, e.g. via a locking mechanism which allows slippage of the suture or loosening the suture.

In this manner the tension or compression of the tissue bulges can be adjustable. This may allow the anchors to be laced together similar to a corset.

The medical apparatus may comprise at least one fastener, in particular a plurality of fasteners. The fastener may be I-shaped or U-shaped and have a first fastening element and a second fastening element which are connected by a connecting element. At least one fastening element may be arranged or arrangeable at least partially circumferentially around the puncturing element or within the puncturing element.

The term fastener in this application may be formed by any kind of thread, suture, and/or staple.

These fasteners enable a quicker and more consistent and uniform connection of the tissue bulges and a facilitated ease of use. The puncturing connection unit may be adapted to deploy the I-shaped fasteners such that the deployed fastener has a straight shape. In the straight shape, the first and second fastening elements may be arranged on opposing sides of the two neighbouring tissue bulges.

The puncturing connection unit may be configured to deploy the U-shaped fasteners such that the first and second fastening elements or a plurality of first and second fastening elements are arranged only on one side of the two neighbouring tissue bulges.

In addition, the length and/or an elasticity of the connecting element between the fasteners may be configured to optimize the strain on the tissue of the stomach wall to provide a sufficiently secure connection without damaging the tissue.

The first fastening element, the second fastening element, and/or the connecting element may be formed monolithically; in particular by a I-shaped or U-shaped wire. This may provide enhanced structural integrity and/or shape retention of the fastener.

The fastening elements may be adapted such that an effective length of the connecting element can be increased or decreased by changing a spacing between the fastening elements, e.g. by moving one fastening element along the connecting element which connects the fastener element and locking the anchor via a locking mechanism. The effective length may relate to the length of the connecting element which extends through the tissue bulges.

In a preferred embodiment, the fastening element is arranged/arrangeable within an inner lumen of the puncturing element. The puncturing element may have a longitudinal slit extending longitudinally along the puncturing element. The slit may be arranged on a radially outward tangential surface with respect to a curvature of the curved puncturing element. Alternatively, the slit may be arranged on a curvilinear radial flank of the curved puncturing element. The slit may be shaped and sized such that the connecting element of the fastener can longitudinally move along a longitudinal direction of the slit. The slit may be connected to an inner lumen of the puncturing element and in particular extend up to a terminal end of the puncturing element forming an opening. The inner lumen may be adapted to receive the fastening element which is shaped and sized to be confined within the inner lumen and cannot cross the slit but may be deployed from the opening at the terminal end.

The puncturing connection unit may have at least one pair of puncturing elements. The puncturing element of a pair may be arranged adjacent each other. In particular, the puncturing elements may extend along the circumferential direction of the sizing tube, preferably parallel to each other. The pair of puncturing elements may have slits which are arranged facing towards each other. The puncturing elements may be arranged distanced from each other such that the connecting element may extend between the puncturing elements when the fastening elements are arranged on or within the puncturing elements. This allows to place I-shaped or U-shaped fasteners with the connecting element having a U-shape in a fast and efficient manner.

The puncturing element(s) may comprise a fastener deploying member which is adapted to be longitudinally advanced within the inner lumen of the puncturing element(s) for pushing/deploying the fastening element(s) from the inner lumen once the tissue bulges were pierced and in particular, when the puncturing element is retracted from the tissue bulges. The fastener deploying member may be formed by an actuatable arcuate piston inside the inner lumen.

The puncturing connection unit may have a hydraulic, pneumatic, magnetic, or mechanic actuation unit for actuating the puncturing element, in particular for advancing and retracting the puncturing element through the tissue bulges.

This enables a simple actuation of the puncturing element. In particular a hydraulic actuation unit can be implemented in a simple manner for advancing and in particular retracting the puncturing element by applying a positive and negative pressure.

A mechanic actuation unit may comprise a bi-stable spring toggle which has two resting positions and which is configured to advance and/or retract the puncturing element when changing between the resting positions. The spring toggle may be actuated by a trigger element such as a pull wire and configured such that a force can be applied to a spring mechanism of the spring toggle.

The medical apparatus may comprise a gripping means, in particular formed by the puncturing connection unit, which is configured to adjoin the two tissue bulges together prior to connecting the tissue bulges, in particular by engaging the tissue bulges from opposing sides, so that they can be connected by the puncturing connection unit.

This allows to adjoin the tissue bulges with a sufficient force via the gripping means to facilitate connecting the tissue bulges together. The gripping means may be actuatable by a hydraulic, pneumatic, magnetic, or mechanic actuation unit. In particular, the actuation unit which drives the puncturing element may also drive the gripping means.

The gripping means may comprise two movable parts or a movable part and a non-movable part for gripping the tissue. The movable part may be adapted to be advanced towards and retracted, in particular in a synchronized manner with the puncturing element, from the non-movable part of the gripping unit to grip and release the tissue. The gripping means may be synchronized to grip the tissue bulges and compress the tissue bulges prior to advancing the puncturing element to facilitate suturing / deployment of fasteners.

The gripping means may comprise a radially collapsible structure which has two arcuate elements which are preferably joined at their ends or at one of their ends. The radially collapsible structure is collapsible by moving both or one of the ends away from each other or relative to the other end. This allows to apply a radially inwardly directed pressure. The pressure may be uniform on the tissue bulges. The radially collapsible structure may be arranged in a longitudinal direction and/or circumferential direction of the sizing tube and adapted such that the radially collapsible structure may radially engage the tissue bulge(s) when it is converted to the collapsed configuration.

The gripping means may comprise at least two rollers, in particular cylindrical rollers which are adapted for contacting the tissue bulges and are actuatable to advance the tissue bulges further into the receiving opening(s). The rollers are preferably arranged parallel to the longitudinal axis and/or adapted to radially engage the tissue bulges. This facilitates positioning an ideal thickness of the tissue bulges within the receiving opening(s).

The puncturing connection unit may comprise the gripping means and/or the puncturing element and the gripping means may be actuatable in a synchronized manner which allows for a more reliable suturing, e.g. by compressing the tissue bulges prior to piercing them with the puncturing element. This may be achieved by a mechanical coupling element which coupled movement of the gripping means and the puncturing element or a control unit which is configured for controlling the gripping means and puncturing element in a synchronized manner.

The gripping means may be configured such that the puncturing element is arranged/arrangeable within the movable/non-movable part of the gripping means, in particular circumferentially enclosed by the movable/non-movable part of the gripping means. This ensures that the puncturing element punctures the tissue bulges at a location which is securely gripped via the movable/non-movable part of the gripping means.

Alternatively, the puncturing connection unit and the gripping means may be actuatable independently from each other.

The medical apparatus may comprise an implantable layer which is pre-loaded or loadable within the inner lumen of the sizing tube. The implantable layer is attachable to the tissue bulges when connecting the two tissue bulges via the puncturing connection unit. The implantable layer may be adapted for confining the two tissue bulges within a partial cross-sectional volume of the inner lumen when suction is applied by the at least one vacuum port, in particular by the implantable layer being connected to twolateral opposing sections of the sizing tube.

The implantable layer may reliably confine the tissue bulges within the partial cross-sectional region of the inner lumen, in particular along the entire length of the receiving opening(s). This arrangement ensures that the tissue bulges cannot be pulled into the at least one vacuum port and potentially clog it. The implantable layer may be adapted such that the tissue bulges are sucked into the receiving opening(s) only up to a pre-determined depth. This may allow controlling the thickness of the tissue bulges at which the suture or the fastener is deployed, resulting in better patient outcomes. In addition, the increased shielding of the implantable layer and/or properties of the implantable layer offer enhanced protection and improved healing, which reduces the criticality of suturing or deploying fasteners through a specific tissue bulge, thickness.

The implantable layer may comprise or consist of (i) a haemostatic component for reducing bleeding, (ii) a drug delivery layer which is adapted for enhancing haemostasis and/or reducing scarring, and/or (iii) a waterproof layer for rendering a connection region of the two tissue bulges less permeable to fluids. The waterproof layer may be formed by a synthetic and/or a biological material.

This implantable layer according to (i) - (iii) may prevent leakage or wound infection and improve wound healing and protection from contaminants. In addition, the implantable layer allows for different thicknesses of tissue bulges to be punctured without exhibiting high sensitivity to excessive penetration depth, thus avoiding adverse effects of less precise suturing and/or fastener deployment.

In particular, the localized drug delivery (ii) by the implantable layer may provide a sustained local release of therapeutic agents directly to a puncture site. The implantable layer may further comprise different/additional of therapeutic agents such as antibiotics, antiseptics, local anaesthetics, anti-inflammatory drugs, or growth factors to improve healing/prevent infection. The implantable layer may comprise or consist of synthetic polymers, such as polyurethanes or polyurethane derivatives, or biological or xenogeneic tissue, such as bovine pericardium, for decreasing fluid permeability of the implantable layer.

As an alternative to the elongated partitioner, the medical apparatus may have a separation member, in particular having a flexible separation fabric, which is deployable laterally outwardly with respect to the sizing tube extending through one tissue receiving opening of the sizing tube for separating the two tissue bulges from each other along the longitudinal direction when applying suction.

The expansion unit may comprise the separation member for separating the tissue bulges. The expansion unit may be adapted for collapsing and deploying the separation member when being converted between the collapsed and expanded configuration.

The separation member may comprise or consist of a flexible separation fabric. This separation fabric may support the formation of two adjacent tissue bulges.

The separation member of the expansion unit may be attached to the expansion unit in a manner to extend along the receiving opening(s), in particular the entire receiving opening(s), in the longitudinal direction. The separation member may have a proximal and distal end which is attached to the elongated element or the inflatable structure of the expansion unit.

The separation member may be movable in the longitudinal direction along the tissue receiving opening. This may allow for sequentially connecting the tissue bulges at the location of the separation member via the puncturing connection unit.

This allows to locally create the two tissue bulges which are separated by the separation member, e.g. instead of being separated via the elongated partitioner. The separation member which is movable in the longitudinal direction can be easily removed from amidst the two tissue bulges, e.g. from a localized suturing/fastening region, once the tissue bulges were connected with each other. In addition, the separation member may be laterally outwardly deployable at a plurality of longitudinal positions of the tissue receiving opening(s) and/or to different degrees of lateral deployment, such that the position/thickness of the tissue bulges can be adjusted by a longitudinal position and/or a lateral outward extension of the separation member. This enables reliable separation of the tissue bulges to ensure that at least two tissue bulges are received within the receiving opening(s), in particular that the dorsal tissue wall and the ventral tissue wall are received in one receiving opening, respectively. This may also allow tailored treatments according to specific indication or a patient-specific anatomy.

The medical apparatus may have an elongated deployment member which is connected or connectable to the separation member and adapted to reversibly deploy the separation member by pivoting the elongated deployment member laterally outwardly. Alternatively, the elongated deployment member may be connected or connectable to the separation member and adapted for reversibly deploying the separation member by longitudinally advancing the elongated deployment member such that a section of the elongated deployment member which is connected to the separation member moves laterally outwardly with respect to the sizing tube.

This allows to easily deploy the separation member while permitting a longitudinal displacement along the receiving opening. In addition, this enables the separation member to prevent entrapment amidst the tissue bulges by being collapsed after deployment.

The separation member may be arranged parallel to the longitudinal direction of the sizing tube. This allows for a simple design of the medical apparatus and deployment within the receiving opening.

The separation member, in particular the flexible separation fabric, may have an essentially triangular shape in a deployed configuration.

The separation member may be connected to the puncturing connection unit such that the separation member and the puncturing connection unit are longitudinally movable in unison or at least in a synchronized manner. Alternatively, the separation member may be movable independently from the puncturing connection unit.

The medical apparatus may comprise a robotic system for carrying out the endoscopic gastric remodelling procedure in a partially autonomous manner, in particular in a fully autonomous manner.

This allows a high level of precision and consistency, improved workflow efficiency, and reduces the required expertise of a clinician.

Another aspect of the invention relates to a method for carrying out an endoscopic gastric remodelling procedure using a medical apparatus, in particular a previously describe medical apparatus. The method comprises the steps of (i) inserting a sizing tube of the medical apparatus perorally across the oesophagus into the stomach of a patient. The method optionally comprises (ii) sealing an outlet and/or an inlet of the stomach using a sealing unit of the medical apparatus and/or (iii) laterally outwardly deploying a separation member through a tissue receiving opening of the sizing tube by pivoting or longitudinally advancing an elongated deployment member. Additionally or alternatively, the method may comprise converting the expansion unit of the medical apparatus for spreading the stomach from a collapsed configuration to an expanded configuration. The method further comprises applying-suction via at least one vacuum port of the sizing tube to form two tissue bulges of the stomach tissue. The method optionally comprises moving a puncturing connection unit of the medical apparatus in the longitudinal direction with respect to the sizing tube. The method comprises connecting the two tissue bulges with each other by puncturing the tissue bulges.

When inserting the sizing tube, the sizing tube may be guided by a medical instrument, in particular an endoscope or a balloon catheter.

Sealing the outlet and/or the inlet of the stomach using the sealing unit may be carried out by arranging the sealing unit at least partially in the outlet and/or the inlet and at least partially inflating the sealing unit.

The sizing tube may be radially expanded after the insertion, in particular by inflating the sizing tube, to increase a cross-sectional dimension of the sizing tube.

A position and/or orientation of the sizing tube in the stomach may be adjusted using articulated segments which form a longitudinal subsection of the medical apparatus, e.g. a medical delivery device, and are connected to the sizing tube. The position and/or orientation of the sizing tube may be adjustable towards the greater curvature of the stomach with respect to a position and/or orientation of.residual components of the medical apparatus.

Further embodiments of the invention and improvement of the described embodiments will become apparent with the following nonlimiting description of the embodiments.

The invention is now described with reference to certain embodiments and figures which show:
Figures 1A - 1E: a longitudinal cross-sectional view of a medical apparatus being inserted into the stomach, sealing the stomach, suturing the stomach, and being removed from the stomach,
Figures 2A and 2B: a perspective view of a medical apparatus which is inserted into a gastric model and applying suction to form two tissue bulges which are received in two tissue receiving openings of a sizing tube,
Figures 3A and 3B: a longitudinal cross-sectional view of a sizing tube which is inserted in a collapsed configuration through the oesophagus into the stomach and transitioning to an expanded configuration,
Figures 4A and 4B: a perspective side view of two embodiments of the sizing tube of the medical apparatus which have an elongated partitioner with two joined ends and one joined end and one free end, respectively,
Figures 5A and 5B: a perspective view of an embodiment of the sizing tube which has an elongated partitioner which has a plurality of separated longitudinal subsections and a separable joined end,
Figures 6A to 6D: a cross-sectional and perspective view of an embodiment of the sizing tube which has an implantable layer which is arranged within an inner lumen of the sizing tube which is connectable to tissue bulges in parallel with connecting the tissue bulges,
Figures 6E and 6F: a perspective view of the connected tissue bulges and the stomach having a connected dorsal and ventral wall after carrying out the steps described in Figs. 6C and 6D,
Figures 7A - 7D: a perspective side view of a first embodiment of a separation member of the medical apparatus which is laterally outwardly deployed by advancing an elongated deployment member in a longitudinal direction of the medical apparatus,
Figures 7E - 7H: a perspective side view of a second embodiment of a separation member of the medical apparatus which is laterally outwardly deployed by advancing an elongated deployment member in a longitudinal direction of the medical apparatus,
Figures 7I - 7K: a perspective side view of a third embodiment of a separation member and a sizing tube of the medical ap-' paratus which is laterally outwardly deployed by pivoting a deployment member laterally outwardly,
Figures 7L and 7M: a perspective side view of a fourth embodiment of a separation member which is laterally outwardly deployable by a deployment member,
Figures 8A to 8D: a perspective view and a cross-sectional view of two embodiments of a sizing tube and a puncturing connection unit of the medical apparatus,
Figures 9A and 9B: a perspective view of a puncturing element and a fastener of the medical apparatus,
Figures 9C and 9D: a partially cut away perspective view of a puncturing connection unit having a gripping means and a fastener arranged around the puncturing element and within the puncturing element, respectively,
Figure 9E: a perspective view of an alternative embodiment of the puncturing element which has a predetermined fracture or deployment section,
Figure 9F: an exploded perspective side view of a puncturing connection unit,
Figures 10A and 10B: a perspective view of a puncturing element having-an inner lumen for receiving a fastener element, a longitudinal slit extending along the inner lumen, and a fastener deployment member,
Figures 10C and 10D: a perspective view and cross-sectional view of the deployed fastener through the tissue bulges without and with an implantable layer of Figs. 6A - 6F,
Figure 10E: a perspective side view of a medical apparatus which has a puncturing connection unit which is translationally movable along a longitudinal direction of a sizing tube, Figures 11A - 11F: a cross-sectional view and a perspective view of a different embodiment of the sizing tube having a guiding feature in an inner lumen,
Figures 12A - 12D: a perspective view of an embodiment of a puncturing connection unit which has longitudinally movable articulated segments which are movable relative to each other in a longitudinal direction of the sizing tube,
Figures 12E and 12F: a schematic view of a sizing tube which is adapted to alternatingly arrange the dorsal and ventral tissue bulges along a longitudinal direction and a puncturing connection unit comprising helical articulated segments,
Figures 12G - 12J: a schematic representation of different mechanic actuation units for actuating the puncturing connection unit,
Figure 13A: a perspective side view of a sizing tube of the medical apparatus which was inserted into stomach tissue and which applies suction to form dorsal and ventral tissue bulges,
Figures 13B and 13C: a perspective side view of a sizing tube of the medical apparatus which has an expansion unit for spreading the stomach in a collapsed and expanded configuration, respectively,
Figures 13D - 13G: a perspective side view of a sizing tube of the medical apparatus which has an expansion unit which was inserted into the stomach and is controlled in a synchronized manner with a fluid supply and/or suction via a vacuum port,
Figures 14A and 14B: a'perspective top and side view of a sizing tube of the medical apparatus which has a perforated barrier,
Figure 14C: a perspective side view of a sizing tube of the medical apparatus comprising an expansion unit which has a separation fabric,
Figures 15A - 15D: a cross-sectional view of different embodiments of the sizing tube having circumferentially differently positioned and dimensioned longitudinal tissue receiving openings,
Figures 16A and 16B: a perspective view of a plurality of pairs and a pair of puncturing elements with longitudinal slits facing towards each other, respectively,
Figures 16C and 16D: a schematic representation of an alternative embodiment of a fastener,
Figures 16E and 16F: perspective views of two tissue bulges which were connected by a plurality of fasteners deployed via puncturing elements of Figs. 16A and 16B,
Figure 16G: a perspective view of a plurality of puncturing elements with different diameters puncturing two tissue bulges,
Figure 16H: a perspective view of two tissue bulges which were connected by a plurality of fasteners deployed via the puncturing elements of Fig. 16G in a zig-zag pattern,
Figures 17A and 17B: a schematic view of an embodiment of a gripping means for gripping the tissue bulges via a radially collapsible structure,
Figure 17C: a schematic view of a different embodiment of a gripping means for gripping the tissue bulges via two actuatable rollers,
Figures 18A and 18B: a perspective side view of an embodiment of the sizing tube of the medical apparatus,
Figures 19A - 19C: a side view and two perspective views of different embodiments of a perforated barrier of a sizing tube,
Figures 20A - 20D: a schematic representation of different embodiments of a medical apparatus which comprises a sizing tube that is movable by an articulated segment(s) of the medical apparatus,
Figures 21A and 21B: a semi-opaque perspective view of two embodiments of the medical.apparatus having a sizing tube with a helical guiding feature and a longitudinal guiding feature, respectively.

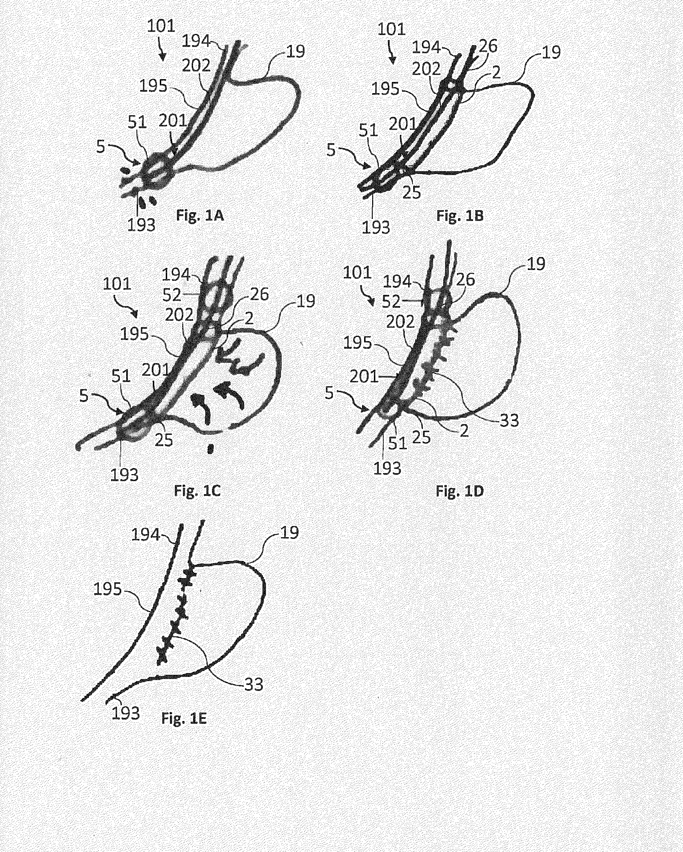

The figures 1A - 1E show a longitudinal cross-sectional view of a medical apparatus 101 being inserted into the stomach of a patient, sealing the stomach, suturing the stomach, and being removed from the stomach.

Figure 1A shows that a medical instrument 201 comprising an endoscope 202 or guidewire is introduced through the oesophagus sphincter which forms an inlet 194 of the stomach along a lesser curvature 195 of the stomach tissue 19 up to the pyloric sphincter which forms an outlet 193 of the stomach. The medical instrument 201 has a sealing unit 5 which comprises a first sealing element 51 formed by an expandable balloon which is used for sealing the outlet 193 of the stomach by inflating the first sealing element 51.

Figure 1B shows that a sizing tube 2 of the medical apparatus 101 is advanced over the medical instrument 201 into the stomach such that the sizing tube 2 extends along the lesser curvature 195 of the stomach tissue 19 between the pylorus and duodenum. The sizing tube 2 has a proximal and distal terminal opening 26, 25 such that it is advanceable over the medical instrument 201.

Figure 1C shows that a second sealing element 52 of the sealing unit 5 formed by a balloon is advanced over the medical instrument 201 and inflated for sealing the inlet 194 of the stomach. Subsequently, a suction is applied by at least one vacuum port (not shown in Figs. 1A - 1E) of the sizing tube 2 indicated by the arrows in Fig. 1C. Based on the sealing of the stomach by the sealing unit 5, the suction is confined to the stomach volume. The applied suction enables a formation of tissue bulges of the dorsal stomach wall and the ventral stomach wall of the stomach tissue 19 which enter the sizing tube 2 through at least one receiving opening.

Figure 1D shows that a puncturing connection unit (not shown in Fig. 1D) of the medical apparatus 101 is used for suturing the two tissue bulges together by deploying a plurality of sutures 33 or fasteners. The sutures create a tube formed by native stomach tissue 19 along the lesser curvature 195 of the stomach which decreases the stomach's volume to a value between 100 - 200 ml, i.e. reducing the stomach's volume to about 15 - 20% in comparison to its original volume.

Figure 1E shows that the medical apparatus 101 was removed from the stomach without leaving any implants other than the sutures 33 or fasteners such that an endosleeve of essentially circular cross-section is formed extending between the oesophagus and the pylorus.

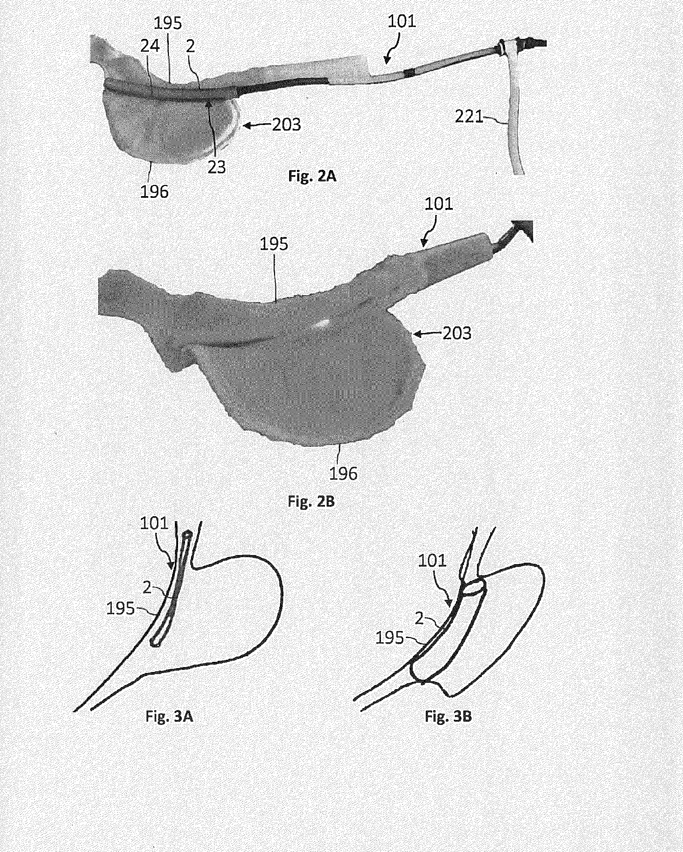

Figures 2A and 2B show a perspective view of a medical apparatus 101 which is inserted into a gastric model 203 of a stomach and applying suction to form two tissue bulges which are received in two tissue receiving openings 23, 24 of a sizing tube 2. A vacuum port of the sizing tube 2 for applying suction is connected to an extracorporeally arrangeable deflation/inflation pump via a supply line 221. The gastric model 203 has a lesser curvature 195 along which the sizing tube 2 is positioned in Fig. 2B and a greater curvature 196. Figure 2B shows that the dorsal and ventral wall of the gastric model 203 are received in two separate receiving openings such that they can connected via a puncture connection unit.

Figures 3A and 3B show a longitudinal cross-sectional view of a sizing tube 2 of a medical apparatus 101 which is inserted in a collapsed configuration through the oesophagus into the stomach adjacent a lesser curvature 195 of the stomach and transitioning to an expanded configuration. The sizing tube 2 is radially expandable along its entire length in an uniform manner by being at least partially inflatable. In this manner, the cross-sectional dimension of the sizing tube 2 can be radially expanded from a value between 6 mm - 16 mm to 20 mm - 27 mm such that the insertion is simplified and the size of the endosleeve can be adjusted to the specific needs of the patient by adjusting the cross-sectional dimension according to the specific anatomy or indication.

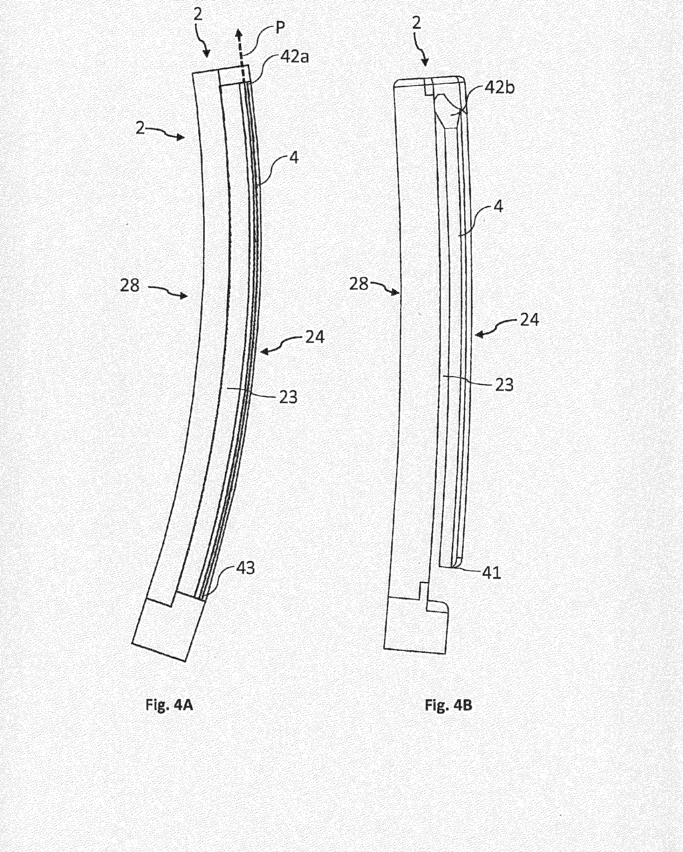

The figures 4A and 4B show a perspective side view of two embodiments of the sizing tube 2 of the medical apparatus which have an elongated partitioner 4 with two joined ends 42, 43 and one joined end 42 and a free end 41, respectively. The sizing tube 2 has a bent shape 28 or is deformable to a bent shape to better conform to a trajectory of the lesser curvature of the stomach. The elongated partitioner 4 is adapted for separating two tissue receiving openings 23, 24 of the sizing tube 2 such that a formation of two tissue bulges by applying suction is possible.

Figure 4A shows that the elongated partitioner 4 has two ends 42a, 43. The distal end 43 is separable from the sizing tube by retracting the entire elongated partitioner 4 with respect to the sizing tube 2 in a proximal direction P.

Figure 4B shows that the free end 41 of the elongated partitioner 4 is arranged at a distal end of the elongated partitioner 4. The proximal end 42b in Fig. 4B forms a monolithically joined end with the sizing tube 2.

This retractable end 43 or free end 41 of the elongated partitioner 4 in Figs. 4A and 4B enable the elongated partitioner 4 to be removed from amidst the tissue bulges within the receiving openings 23, 24 after connecting the tissue bulges has been achieved, in particular by suturing or deploying fasteners. The design of Fig. 4A allows that the elongated partitioner 4 can be retracted in the proximal direction from between the tissue bulges such that the sizing tube 2 can then be removed in a simple manner. The design of Fig. 4B enables removal of the elongated partitioner 4 in conjunction with retraction of the sizing tube 2 such that the elongated partitioner 4 slides off from a region amidst the connected tissue bulges.

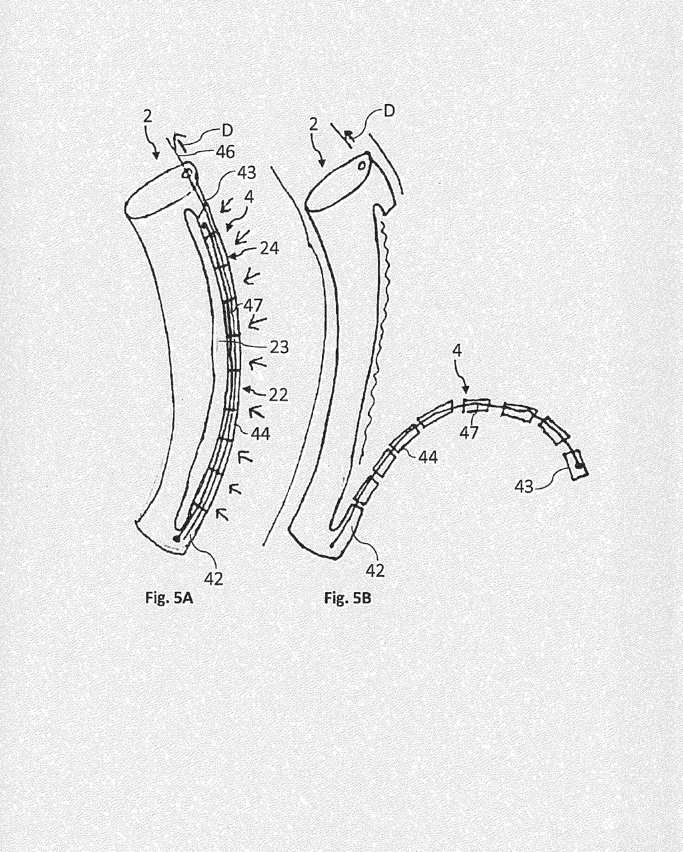

Figures 5A and 5B show a perspective view of an embodiment of the sizing tube 2 which has an elongated partitioner 4 which has a plurality of separated longitudinal subsections formed by tubular elements 44 and a separable distal end 43. The elongated partitioner 4 is positioned to separate two tissue receiving openings 22, 23 of the sizing tube 2 from each other. The elongated partitioner 4 is convertible between a non-flexible state having a bent shape shown in Fig. 5A and a more flexible state shown in Fig. 5B by pulling a conversion element formed by a pull wire 46 in a distal direction D such that the tubular elements 44 are freely movably connected to each other. The tubular elements 44 are still connected via a connecting wire 47 which is connected to the sizing tube 2 such that the tubular elements 44 are retrievable from the stomach together with the sizing tube 43. Even though Figs. 5A and 5B show removing the pull wire 46 in a distal direction, the pull wire 46 may alternatively be removable in a proximal direction to release the end 43.

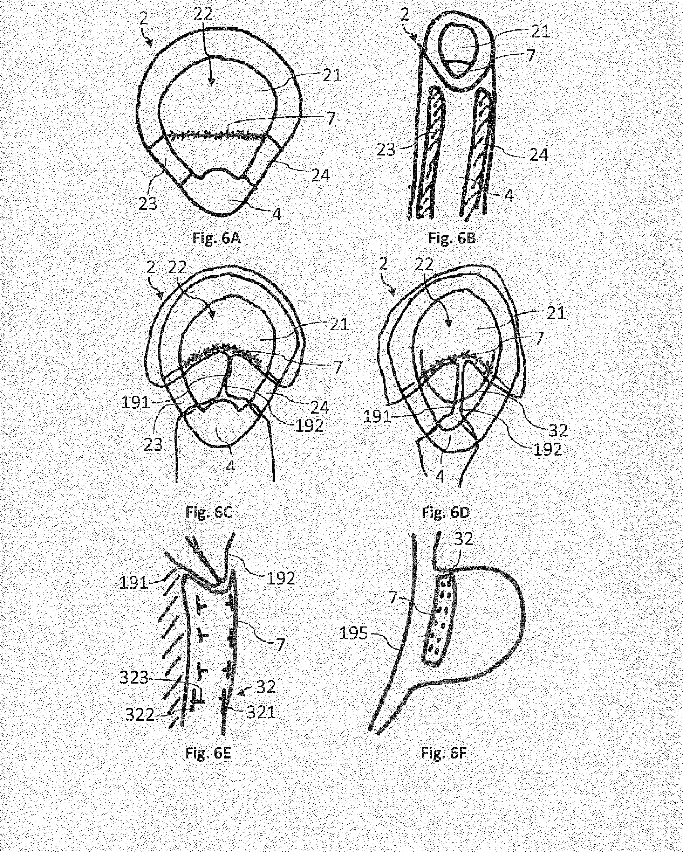

Figures 6A - 6D show a cross-sectional and perspective view of an embodiment of the sizing tube 2 which has an implantable layer 7 within an inner lumen 21 of the sizing tube 2 which is connectable to tissue bulges 191, 192 at the same time as with connecting the tissue bulges 191, 192 with each other. The implantable layer 7 is arranged between a vacuum port 22 of the sizing tube 2 and two receiving openings 23, 24 for receiving the tissue bulges 191, 192. The receiving openings 23, 24 are separated from each other by an elongated partitioner 4. The cross-section of the sizing tube 2 and the elongated partitioner 4 form a wedge shape with the elongated partitioner 4 forming the tapered end to facilitate the formation of the ventral and dorsal bulges 23, 24. The elongated partitioner 4 and/or the implantable layer 7 may be sized and arranged in such a manner that the serosa of the tissue bulges 191, 192 may be punctured by a puncturing connection unit (not shownin Figs. 6A - 6D). The receiving openings 191, 192 are further arranged such that the first receiving opening 23 receives a part of the ventral tissue wall and the second receiving opening 24 receives a part of the dorsal tissue wall. The elongated partitioner 4, the vac-, uum port 22 and the receiving openings 23, 24 may extend over the majority of a longitudinal length of the sizing tube 2 or even the entire longitudinal length..

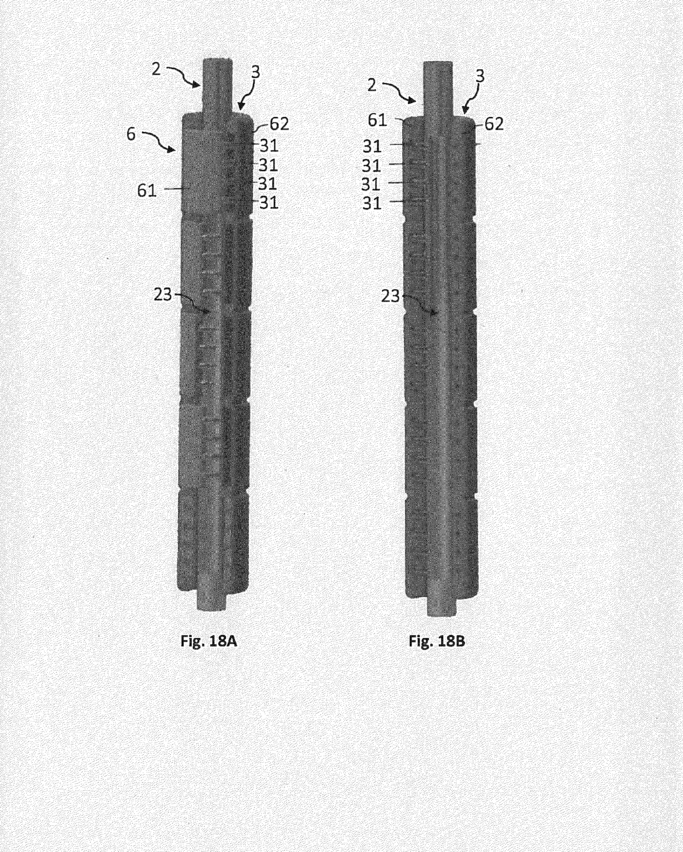

The implantable layer 7 may be at least partially permeable to applied suction such that the tissue bulges 191, 192 can be sucked into the receiving openings 23, 24. The implantable layer 7 may comprise holes, a mesh structure, or cut-outs, in particular in regions of the implantable layer 7 which are not to be punctured by a puncturing element (similar to Figs. 18A - 18C). The implantable layer 7 may be formed by a woven or microporous fabric or comprise a specific cutout pattern to enable permeability to applied suction. The implantable layer 7 may comprise expanded polytetrafluoroethylene and/or polyurethane.

Figure 6C shows that the tissue bulges 191, 192 are sucked through the receiving openings 23, 24 in such a manner that they contact the implantable layer 7 and deform it.

Figure 6D shows deployment of a suture or fastener 32 through the implantable layer 7 and the tissue bulges 191, 192 for fastening them together. Subsequently, the elongated partitioner 4 may be removed from between the tissue bulges 191, 192 as previously described or e.g. by being self-dissolving, soluble, or bioresorbable.

Figures 6E and 6F show a perspective view of the connected tissue bulges 191, 192 and the stomach having a connected dorsal and ventral wall covered by the implantable layer 7 after carrying out the steps described in Figs. 6C and 6D. The implantable layer 7 is connected via a plurality of fasteners 32 which have an I-shape and comprise a connecting element 323 which is provided on both sides with a fastening element 321, 321 which extends essentially perpendicularly to the connecting element 323. The implantable layer 7 is impermeable/less permeable to fluids, at least at puncture sites at which fasteners 32 were deployed such that the risk of infection can be reduced. The implanted implantable layer 7 in Figs. 6E and 6F isolates the punctured sections of the tissue bulges from fluid, thus eliminating the necessity of the tissue bulges to independently establish a fluid impermeable bond. The implantable layer 7 further or alternatively has a haemostatic fabric which reduces bleeding from the puncture wounds. Figure 6F shows that the final endosleeve is formed by a tubular section between a lesser curvature 195 of the stomach and the connected dorsal and ventral wall.

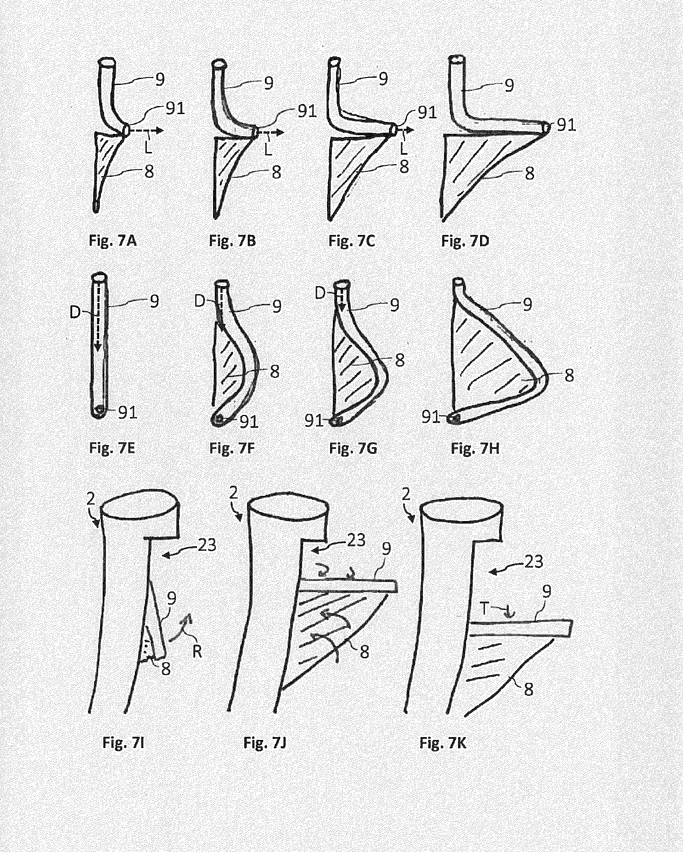

Figures 7A - 7D show a perspective side view of a first embodiment of a separation member 8 for separation of the tissue bulges as an alternative to the previously described elongated partitioner which is laterally outwardly deployed by advancing an elongated deployment member 9. A distal tip 91 of the deployment member 9 which is formed by a flexible push rod is attached to the separation member 8 such that advancing the distal tip 91 in a distal direction is redirected in a laterally outward direction L. Based on the laterally outward movement a triangular flexible separation fabric which forms the separation member8 can be deployed.

Figures 7E - 7H show a perspective side view of a second embodiment of a separation member 8 which is laterally outwardly deployable by advancing an elongated deployment member 9 in a longitudinal direction of the medical apparatus. A distal tip 91 of the deployment member 9 is connected to the sizing tube such that advancing a proximal end of the deployment member 9 in a distal direction D bends the deployment member 9 to an arcuate shape which deploys the separation member 8 which is formed by a flexible separation fabric. The separation member 8 is radially attached or connected to the deployment member 9 along an entire longitudinal dimension of the separation member 8.

Figures 7I - 7K show a perspective side view of a third embodiment of a separation member 8 and a sizing tube 2 of the medical apparatus which is laterally outwardly deployed by pivoting a deployment member 9 laterally outwardly in a direction R away from,a longitudinal axis of the sizing tube 2. Figures 7I - 7K show that the separation member 8 is deployed within the tissue receiving opening 23 to separate the tissue receiving opening 23 into two separated sections on either side of the separation member 8 such that the formation of two tissue bulges can be facilitated. The deployment member 8 of Figs. 7A - 7H.may also be arranged and configured to separate sections of the receiving opening or the entire receiving openings.

The separation member 8 and deployment member 9 in the embodiments of Figs. 7A - 7K may be movable in unison together along the tissue receiving opening 23 of the sizing tube 2 such that the spatial position of tissue bulge formation is adjustable. This allows to move the separation member 8 away from sections of tissue bulges which have already been connected until the endosleeve surgery is completed. Subsequently, the separation member 8 can be moved to a collapsed configuration by pivoting the deployment member 9 or retracting the deployment member 9 in a proximal direction.

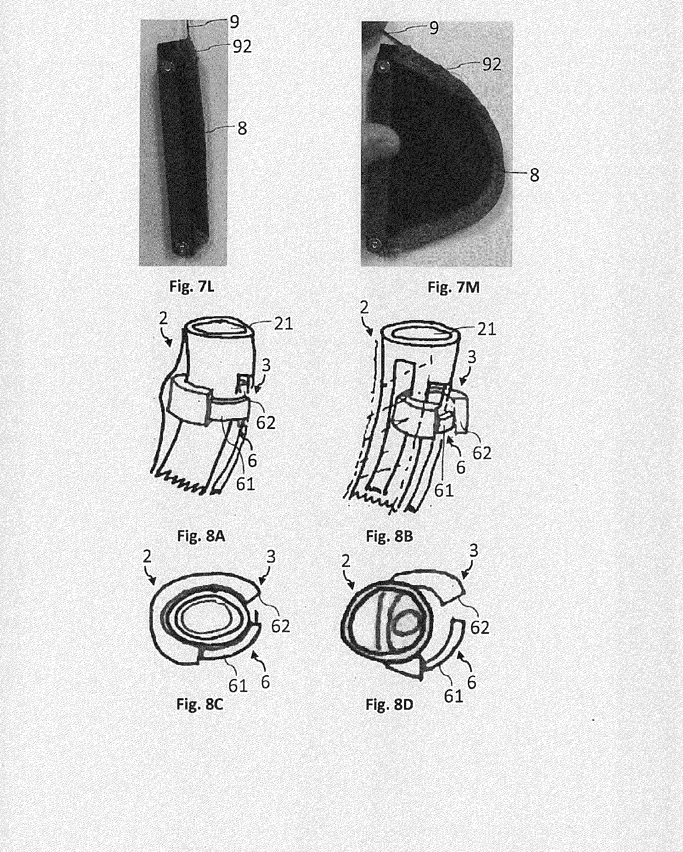

Figures 7L and 7M show a perspective side view of a fourth embodiment of a separation member 7 which is laterally outwardly deployable by a deployment member 9. The deployment member 9 is arranged within a flexible guide sleeve 92 which confines the movement of the deployment member 9 in a lateral and longitudinal direction. By advancing the deployment member in a distal direction, the deployment member 9 is deformed within the guide sleeve 92 to a curvature to deploy the separation member 8 which is formed by a flexible separation fabric which is connected along its longitudinal direction to the guide sleeve 92.

Figures 8A to 8D show a perspective view and a cross-sectional view of two embodiments of a.sizing tube 2 and a puncturing connection unit 3 of the medical apparatus. The puncturing connection unit 3 has an annular shape and has a gripping means 6. The gripping means 6 are formed by a movable part formed by a hammer 61 which is adapted to be advanced back and forth in a circumferential direction towards/away from a second part formed by an anvil 62 to deploy a suture or a fastener through tissue bulges. The puncturing connection unit 3 may be configured such that the hammer 61 is moved in the circumferential direction, while the anvil 62 is rotated in the opposite direction, such that the hammer 61 and anvil 62 meet in the middle of a receiving opening. This may be achieved by the puncturing connection unit 3, or at least the anvil 62, being rotatably arranged with respect to the sizing tube 2.

The puncturing connection unit 3 is configured to be moved in the longitudinal direction of the sizing tube 2, in particular by an actuator of the medical apparatus, such that the tissue bulges can be sequentially connected.

In Figs. 8A and 8C the puncturing connection unit is arranged circumferentially around an outer mantle surface of the sizing tube 2. The sizing tube 2 and the puncturing connection unit 3 is enclosed by a cover along a part of the circumference of the sizing tube. The cover.extends along the sizing tube 2 such that the puncturing connection unit 3 is movable underneath the cover. The cover may optionally form an inflatable balloon, e.g. to radially expand the sizing tube 2.

In Figs. 8B and 8D, the puncturing connection unit is arranged within longitudinal openings of the sizing tube 2 such that the puncturing connection unit 3 is partially arranged within an inner lumen 21 of the sizing tube 2 in Figs. 8B and 8D.

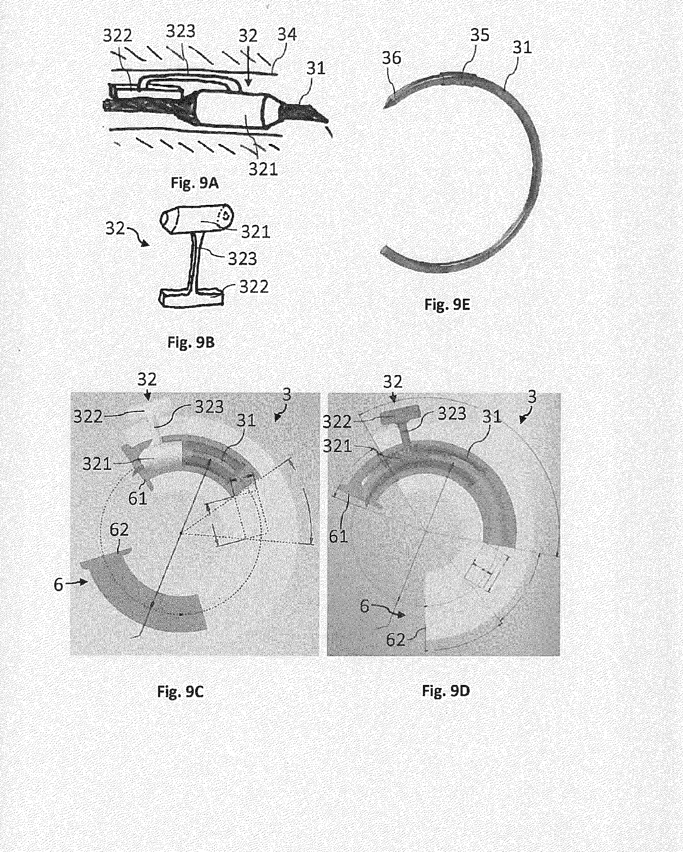

Figures 9A and 9B show a perspective view of a puncturing element 31 and a fastener 32 of the puncturing connection unit 3 (see Figs. 9C and 9D). The fastener 32 has a first fastening element 321 which has a cylindrical lumen and is arrangeable circumferentially around the puncturing element 31 such that it can be advanced through the tissue bulges together with the puncturing element 31. The first fastening element 321 is connected via a flexible connecting element 323 to a second fastening element 322 of the fastener 32 such that the second fastening element 322 can be arranged adjacent the puncturing element 31 by the connecting element 323 being deformed to a curvature. This allows the fastener 32 to be easily mounted on the puncturing element 31 and to be arranged in a longitudinal guide 34 together with the puncturing element 31.

Figures 9C and 9D show a partially cut away perspective view .of a puncturing connection unit 3 having a gripping means 6 for gripping the tissue bulges and a fastener 32 arranged around the puncturing element 31 and within the puncturing element 31, respectively. The puncturing element 31 is formed by an arcuate needle which is operable by hydraulicly operated pistons. The gripping means 6 are formed by two arcuate parts 61, 62 which may be movable towards each other.

In a preferred embodiment, the first part 61 of the gripping means 6 is movable with respect to the second part 62, in particular in unison with the puncturing element 31, while the second part 62 is non-movable. The puncturing element 31 is arranged within the first part 61 which facilitates reliably gripping the tissue bulges and advancing the puncturing element 31 through the section gripped by the gripping means 6. The puncturing element 31 is circumferentially surrounded by the first part 61 of the gripping means 6 during/prior to puncturing the tissue bulges. Alternatively, the arcuate parts 61, 62 may be configured to be movable towards and apart from each other, in particular by a spring mechanism.

Figures 9C and 9D show that the fastener 32 has a first and second fastening.element 321, 322 which are arranged perpendicular to a connecting element 323 of the fastener 32 to form an I-shape. The first fastening element 321 in Fig. 9C is arranged circumferentially surrounding the puncturing element 31. The first fastening element 321 in Fig. 9D is arranged within an inner lumen of the puncturing element 31. Both embodiments allow the fastener 32 to be advanced through the tissue bulges via the puncturing element 31. This enables placement of the first fastening element 321 on an entry side of the tissue bulges and a second fastening element 322 on an exit side of the tissue bulges while a connecting element 323 of the fastening elements 321, 321 extends through the tissue bulges.

Figure 9E shows a perspective view of an alternative embodiment of the puncturing element, 31 which has a predetermined fracture or deployment section 35. This allows a distal tip 36 of the puncturing element 31 to be deployed in a similar manner to the previously described first fastening element 321 (see Figs. 9A - 9D). The distal tip 36 may also be connected via a connecting element to a second fastening element which is arranged within the puncturing element 31 to be deployed in a similar manner to the fastener 32.

The puncturing connection unit 3 for deploying the fasteners 32 or distal tips 36 of puncturing elements 31 may comprise a reloading or replenishing reservoir/magazine of fasteners 32 or distal tips 36 such that the puncturing element 31 can be reloaded/replenished for deploying further fasteners 32 or distal tips 36.

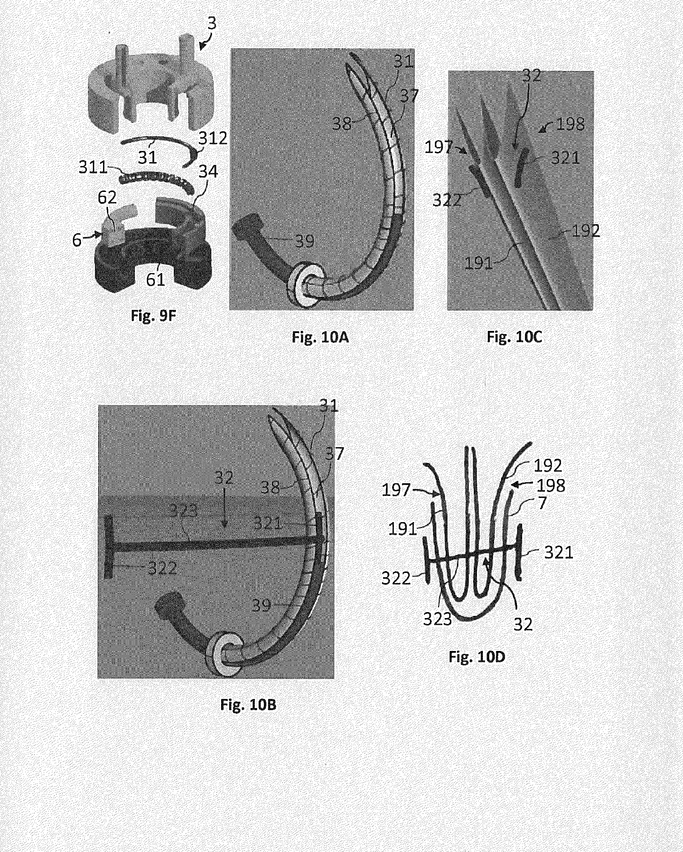

Figure 9F shows an exploded perspective view of a puncturing connection unit 3 which has a puncturing element 31 formed by an arcuate needle which is guided along a circular longitudinal guide 34 which is arranged within a gripping means 6 formed by two parts 61, 62. A spring mechanism, in particular comprising a helical spring 311 and a radial protrusion 312 on the puncturing element 31, is adapted to move the two parts 61, 62 in a circumferential direction away from each other. This allows the two parts 61, 62 to be actuated simultaneously for gripping the tissue bulges and advancing the puncturing element 31 through the tissue bulges. The spring mechanism is further actuatable such that the helical spring 311 is collapsed and the two parts 61, 62 move towards each other, e.g. via actuatable tendons. The guide 34 allows to drive the puncturing element and prevents out of plane movement of the puncturing element and the spring mechanism. This simplifies the actuation of the gripping means 6 and the puncturing element 31 and allows for a synchronized movement.

Figures 10A and 10B show a perspective view of a puncturing element 31 which has an inner lumen 37 for receiving a fastener element 321, a longitudinal slit 38 extending along the inner lumen 37, and a fastener deployment member 39. The puncturing element is formed by an arcuate hollow needle. The longitudinal slit 38 is arranged on a lateral surface of the puncturing element 31. Alternatively, the longitudinal slit 38 may be arranged perpendicular to the curvature of the puncturing element 31. The fastener deployment member 39 comprises actuatable piston which may be actuated by tensioning a spring mechanism for deploying the fastener 32 (not shown in Figs. 10A and 10B). The fastener 32 comprises the first fastening element 321, a second fastening element 322, and a connecting element 323 which form an I-shape or a U-shape.

This allows to deploy the fastener 32 once the puncturing element 31 was advanced through the tissue bulges such that a connecting element 323 of the fastener 32 is deployed through the tissue bulges with the fastening elements 321, 322 deployed on both sides of the tissue bulges. When deploying the fastener 32, the connecting element 323 moves along the slit 38 driven by the fastener deployment member 39 while the first fastening element 321 remains within the puncturing element 31. The fastener 32 can be deployed by the fastening element 321 being released from a terminal end of the puncturing element 31.

The figures 10C and 10D show a perspective view and cross-sectional view of the deployed fastener 32. through the tissue bulges 191, 192 without and with an implantable layer 7 of Figs. 6A - 6F. This allows to place a first fastening element 321 on an entry side 197 of the tissue bulge 191 and a second fastening element 322 on an exit side 198 of the tissue bulge 192 while a connecting element 323 of the fastening elements 321, 321 extends through the tissue bulges 191, 192.

Figure 10D shows that the entry side 197 and the exit side 198 are covered by the implantable layer 7 such that leakage can be reduced, and haemostasis at a puncture site can be promoted.

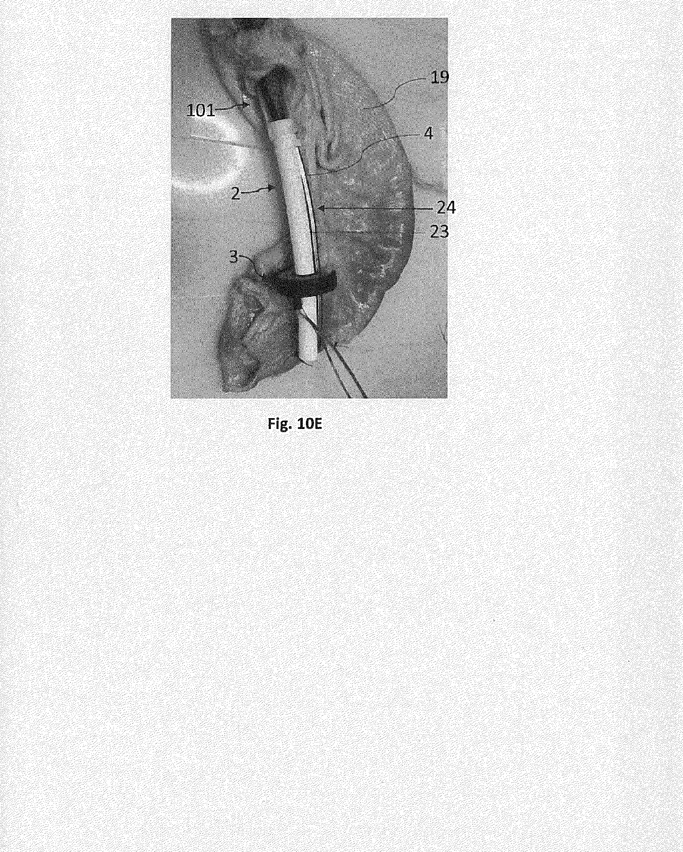

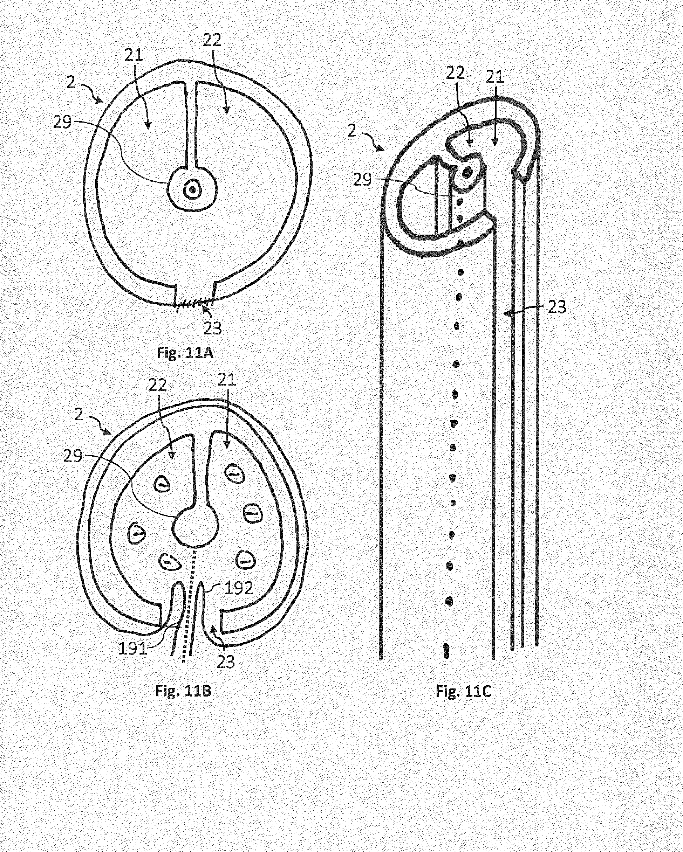

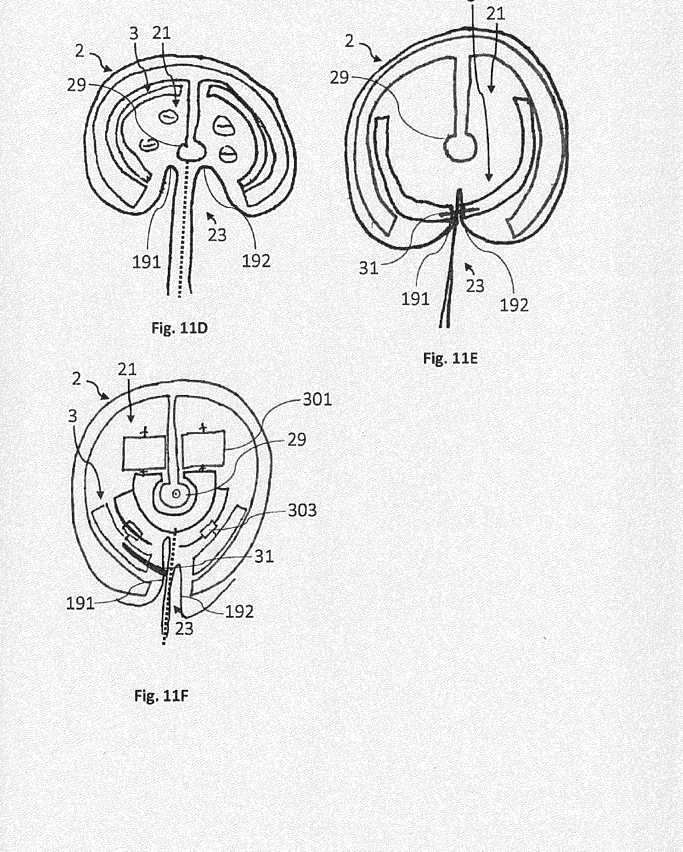

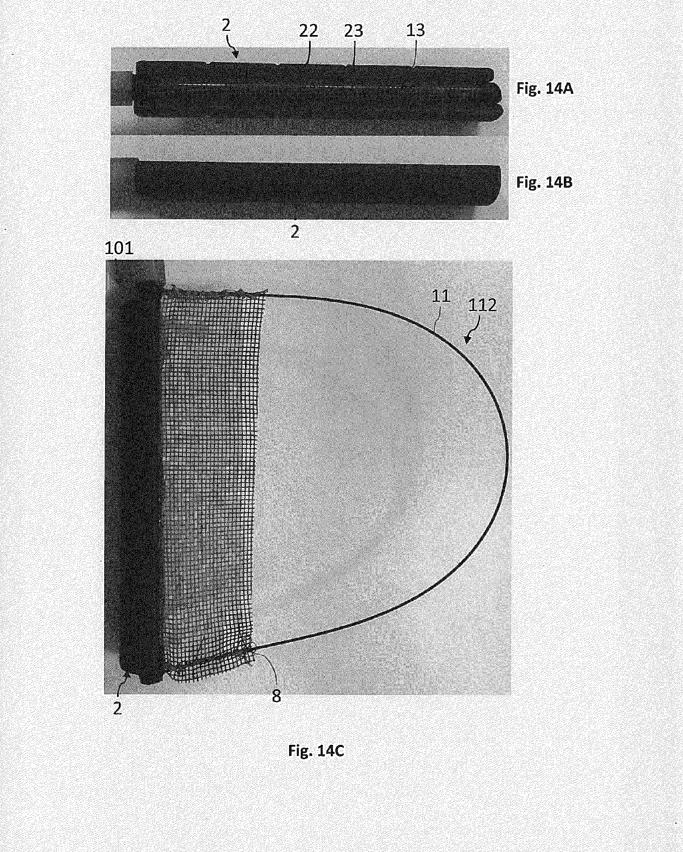

Figure 10E shows a perspective side view of a medical apparatus 101 which has a puncturing connection unit 3 which is translationally movable along a longitudinal direction of a sizing tube 2. When suction is applied by a vacuum port of the sizing tube 2 the stomach tissue 19 forms a dorsal and ventral tissue bulge which are received within two tissue receiving openings 23, 24 of the sizing tube 2 which are separated by an elongated partitioner 4. This allows the puncturing connection unit 3 to deploy previously described fasteners or sutures successively through dorsal and ventral tissue bulges of the stomach tissue 19 successively by moving along the tissue receiving openings 23, 24. Figures 11A - 11F show a cross-sectional view and a perspective view of a different embodiment of the sizing tube 2 having a guiding feature 29 for guiding a movement of a puncturing connection unit (see Fig. 11F) in a longitudinal direction in an inner lumen 21 of the sizing tube 2. The guiding feature 29 is formed by a guide rail which has two undercuts which extends along a longitudinal direction of the sizing tube 2. Figure 11B shows that a negative pressure via a vacuum port 22 can be applied within the inner lumen 21 such that two tissue bulges 191, 192 from the dorsal and ventral stomach wall may be received within a tissue receiving opening 23 which extends along the longitudinal direction. The sizing tube 2 may separate the receiving opening 23 into two openings/sections by an elongated partitioner 4 (see e.g. Figs. 4A and 4B), a separation member 8 . (see Figs. 7A - 7M), or a an expansion unit 11 (see Fig. 14C) indicated by the dashed line in Figs. 11B, 11D and 11F. This may facilitate the formation of the two tissue bulges 191, 192.

Figures 11D and 11E show that a puncturing connection unit 3 may be arranged within the inner lumen 21 which has a gripping unit and a puncturing element 31 for connecting the tissue bulges 191, 192 by deploying a suture or a fastener.

The puncturing connection unit 3 in Fig 11F has a longitudinal translation unit formed by two cylindrical rollers 301 which are adapted for translationally moving the puncturing connection unit 3 along the longitudinal direction. The puncturing connection unit 3 in Fig. 11F further has an actuation mechanism 303 for advancing the puncturing element 31 and the gripping unit for deploying the suture/fastener through the tissue bulges 191, 192 formed within the receiving opening 23. The actuation mechanism 303 is preferably hydraulically actuated.

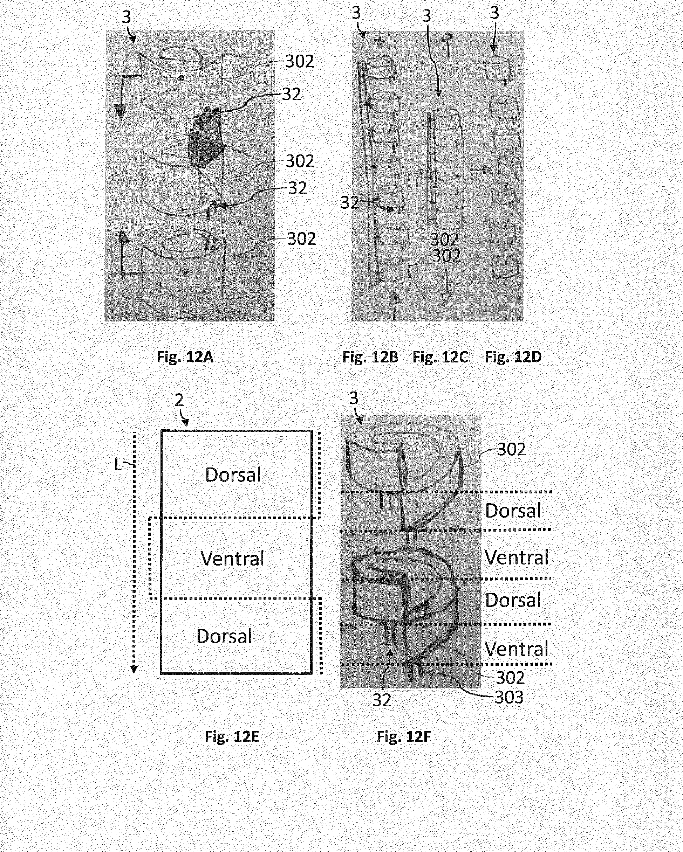

Figures 12A to 12D show a perspective view of an embodiment of the puncturing connection unit 3 which has a plurality of segments 302 which are movable relative to each other in a longitudinal direction of the sizing tube. The sizing tube may have a plurality of receiving openings which are adapted for receiving several longitudinally separated tissue bulges of the dorsal and ventral wall sections. This allows the segments 302 to deploy a fastener 32 to connect the tissue bulges by being moved towards each other. An elongated partitioner or a separation member may extend longitudinally through the segments 302 to facilitate the formation of the two tissue bulges within the tissue receiving opening(s).

Figures 12B - 12D show that the segments 302 may be configured such that the segments 302 are movable toward each other by compressing the segments 302 in the longitudinal direction such that the tips of the fasteners 32 are bent and the fasteners 32 can be deployed to connect the tissue bulges. Subsequently, the segments 302 may be moved apart from each other to release the tissue bulges and fasteners 32 as shown in Fig. 12D.
Figures 12E and 12F show a schematic view of a sizing tube 2 which is adapted to alternatingly arrange the dorsal and ventral tissue bulges along a longitudinal direction L and a puncturing connection unit 3 comprising helical segments 302. The helical segments 302 form only one helical turn which is correspondingly formed for receiving the dorsal or ventral wall tissue bulge in an upper section and the other of the dorsal or ventral wall tissue bulge in the lower section.

Alternatingly arranging the dorsal and ventral tissue bulges may be achieved by arranging the tissue receiving openings in a zig-zag pattern or alternatively arranging the elongated partitioner or separation member in a zig-zag pattern. This allows to arrange a dorsal tissue bulge and a ventral tissue bulge adjacent each other between the segments 302. Each segment 302 has a first fastener 32 at its bottom surface of the upper section and a second fastener 303 at its bottom surface of a lower section. This allows to place more fasteners for connecting the dorsal and ventral tissue bulges by each fastener 32 being deployable through separate dorsal and ventral tissue bulges in an alternating manner. Tips of the fasteners 32, 303 can be bent and deployed by moving the single helix segments 302 towards each other, in particular by longitudinal compression in a similar manner as described in Figs. 12A - 12D.

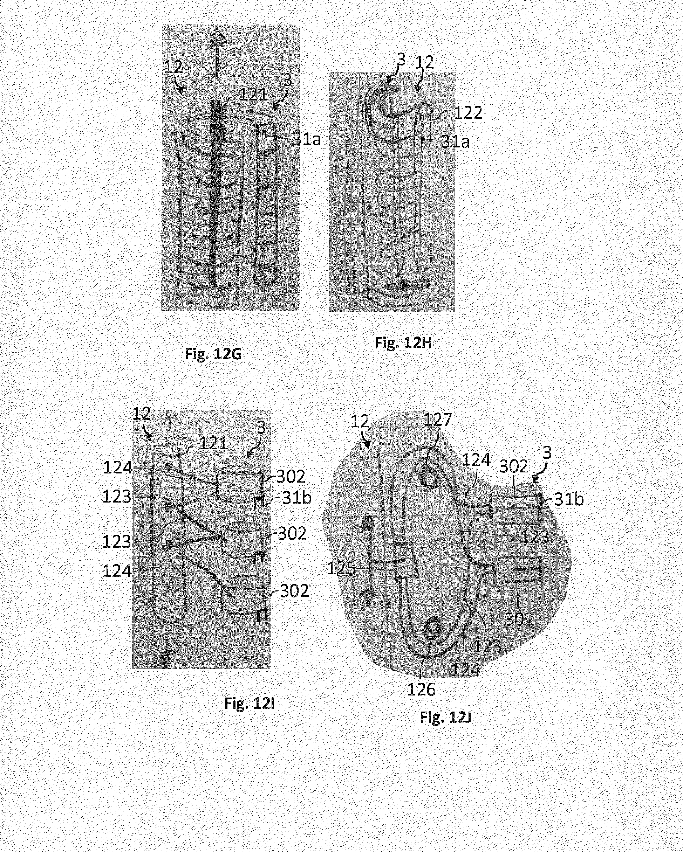

Figures 12G to 12J show schematic representation of different mechanical actuation units 12 of the puncturing connection unit 3 by advancing and/or retracting a puncturing element 31. The mechanical actuation units 12 may be arranged within a sizing tube of the medical apparatus and be adapted to be movable with respect to the sizing tube.

Figure 12G shows a longitudinally translationally movable actuation rod 121 of the mechanical actuation unit 12 which is adapted for frictionally engaging a plurality of puncturing elements 31a of a puncturing connection unit 3 which are formed by arcuate needles. When the actuation rod 121 is advanced within an inner lumen of the sizing tube to the puncturing connection unit 3, the actuation rod 121 can be rotated to frictionally drive a rotational movement of the circumferentially movable puncturing elements 31a in a circumferential direction of the sizing tube to connect two tissue bulges within a receiving opening.

Figure 12H shows a cylindrical mechanical .actuation unit 12 which comprises a radially inwardly extending protrusion 122 which extends longitudinally along an inner surface of the cylindrical mechanical actuation unit 12. The protrusion 122 is configured for engaging a plurality of puncturing elements 31a of a puncturing connection unit 3, in particular only at a proximal end of the puncturing elements 31a and for providing a motive force to the puncturing elements to simultaneously drive the.puncturing elements 31a in a circumferential direction with respect to the sizing tube.

Figure 12I shows an actuation unit 12 which has an actuation rod 121 which is movable in the longitudinal direction for axially actuating a puncturing elements 31b, e.g. to drive a deployment of fasteners as described in Figs. 12A - 12F. The puncturing connection unit 3 in Fig. 12I comprises a plurality of articulated segments 302 which are adapted for deploying the fasteners by puncturing dorsal and ventral tissue bulges together which are arrangeable between the articulated segments 302. Each articulated segment 302 is connected via a first and second tendon 123, 124 to the actuation rod 121. The tendons 123, 124 are coupled to the actuation rod 121 in such a manner that a unidirectional movement of the actuation rod 121, e.g. in a distal or proximal direction, pulls the antagonistic first tendons 123 while releasing the negative second tendons 124 such that the articulated segments 302 move towards each other for suturing. Moving the actuation rod 121 in an opposing unidirectional movement, e.g. the other distal or proximal direction, pulls the second tendons 124 and releases the first tendons to move the articulated segments 302 apart from each other.

Figure 12J shows a mechanical actuation unit 12 which has a longitudinal slider 125 or a push rod. The puncturing connection unit 3 has two articulated segments 302 which are connected to the longitudinal slider 125 via a first and a second tendon 123, 124, respectively.

The antagonistic first tendons 123 and the negative second tendons 124 of the articulated segments 302 are each guided around a proximal and distal tendon guide 126, 127 which may be formed by pulleys, respectively. The slider 125 is adapted such that a unidirectional displacement of the longitudinal slider 125, e.g. by sliding along a guide rail in a proximal or distal direction, pulls the antagonistic first tendons 123 and releases the negative second tendons 124. This moves the articulated segments 302 towards each other to actuate a puncturing element 31b of the puncturing connection unit 3 through the tissue bulges and deploy a suture or a fastener. An opposing unidirectional displacement of the slider 125 instead pulls the negative second tendons 124 and releases the antagonistic first tendons 123 via the pulleys to move the articulated segments 302 apart from each other.

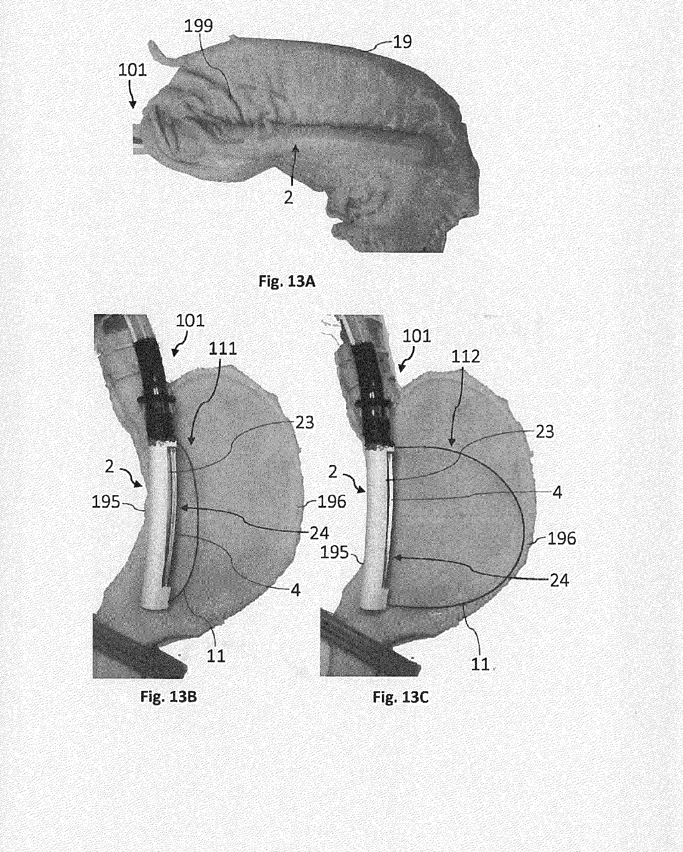

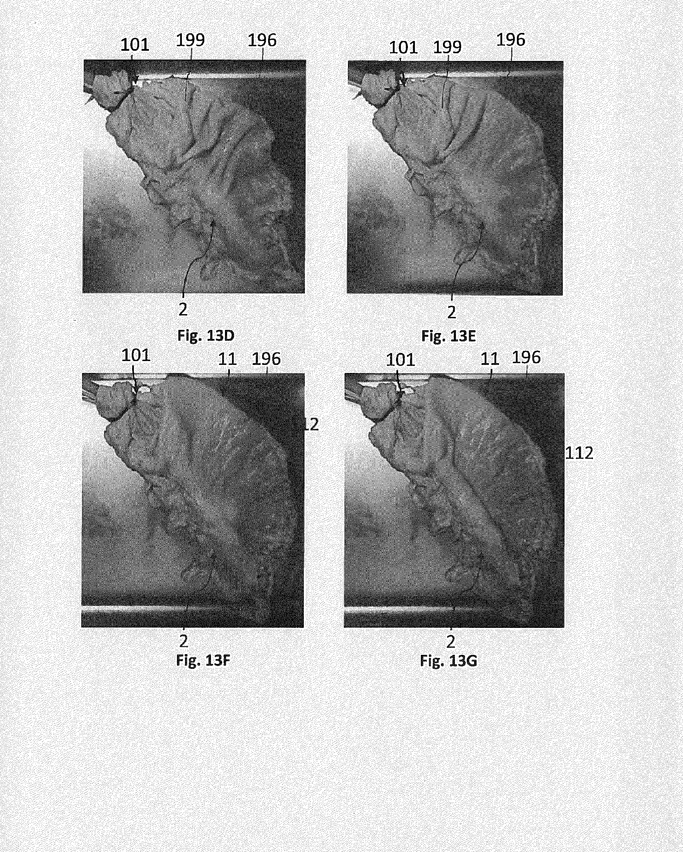

Figure 13A shows a perspective side view of a sizing tube 2 of the medical apparatus 101 which was inserted into stomach tissue 19 and which applies suction to form dorsal and ventral tissue bulges. A vacuum port of the sizing tube supplies the suction along the entire receiving opening(s) of the sizing tube, such that the tissue bulges are received in the sizing tube and can be connected via a puncturing connection unit. Figure 13A shows that the applied suction may result in gastric folds. 199 of the stomach tissue19, in particular in a region in proximity of the oesophageal sphincter, e.g. in the cardia, cardial notch, or fundus region of the stomach. These gastric folds 199 may result in clogging of the sizing tube 2 and/or improper capture of the tissue bulges which may negatively impact the suturing of the tissue bulges. Most importantly, the formation of gastric folds 199 may impair suturing or fastener deployment. In particular, the gastric folds 199 may result in sutures or fasteners being improperly placed, in insufficient numbers per section of stomach tissue 19, or in placement at an insufficient depth. The gastric folds 199 may even result in suture/fastener failure due to tissue apposition. This may further result in adverse surgical outcomes such as gastric leakage, infection, and/or delayed healing. Most importantly, this reduces leakage between sections of the stomach that are separated by the sutures/fasteners which would negatively impact the surgical outcome, in particular regarding weight loss.

Figures 13B and 13C show a perspective side view of a sizing tube 2 of the medical apparatus 101 which has an expansion unit 11 for spreading the stomach assuming a collapsed configuration 111 and an expanded configuration 112, respectively. The expansion unit 11 comprises a nitinol wire which has a distal end which is fixedly connected to a distal end of the sizing tube 2. The expansion unit 11 is convertible between the collapsed configuration 111 and the expanded configuration 112 by advancing/retracting a proximal end of the nitinol wire. In the collapsed configuration 111, the nitinol wire longitudinally extends along two tissue receiving openings 23, 24 of the sizing tube 2 which are separated by an elongated partitioner 4. In the collapsed configuration 111, the nitinol wire of the expansion unit 11 has a first curvature which is lower than a second curvature in the expanded configuration 112 once the nitinol wire was advanced. In the expanded configuration 112, the nitinol wire engages a greater curvature 196 of the stomach tissue and thereby stretches the stomach tissue and urges the sizing tube 2 towards a lesser curvature 195 of the stomach tissue. This enables the formation of gastric folds 199 (see Figs. 13A and 13D - 13G) can be reduced/prevented. This embodiment further provides a uniform pressure distribution for stretching the stomach tissue further along the greater curvature 196 via the nitinol wire such that uniform ventral and dorsal tissue bulges may be formed and received within the tissue receiving openings 23, 24.

Figures 13D - 13G show a perspective side view of a sizing tube 2 of the medical apparatus 101 which comprises the expansion unit 11 which was inserted into the stomach and is controlled in a synchronized manner with a fluid supply and/or suction via a vacuum port. Figures 13D and 13E show that the stomach tissue forms a plurality of gastric folds 199 which complicates sufficiently connecting dorsal and ventral tissue bulges. Figures 13E - 13G show that the expansion unit 11 is successively converted to an expanded configuration 112 to stretch the stomach tissue by engaging a greater curvature 196 of the stomach and remove the gastric folds 199. In conjunction, fluid is supplied to expand a volume of the stomach to facilitate deployment of the expansion unit 11 and reliably remove the gastric folds 199. The fluid supply via a vacuum port may be configured for continuously supplying fluid when converting the expansion unit 11 from the collapsed configuration to the expanded configuration 112. The fluid may be supplied in a synchronized manner with the conversion of the expansion unit 11 such that the volume of the stomach receives fluid inflow which facilitates successive deployment of the expansion unit 11. After completely deploying the expansion unit, in particular without fully inflating the stomach, a suction pressure may be applied to the stomach to remove the fluid again. Once the gastric folds 199 have been removed in this manner, the surgical procedure for connecting the dorsal and ventral tissue bulges can be carried out in a more reliable manner. Figure 13G shows that the tissue bulges which are formed by the stomach tissue and received within the tissue receiving opening(s) are more uniform without the gastric folds 199 once suction is applied.

In a preferred embodiment, the medical apparatus 101 comprises a control unit which is adapted for operating the expansion unit 11 in a synchronized manner with a fluid supply and/or suction of a vacuum port of the sizing tube 2.

Figures 14A and 14B show a perspective top and side view of a sizing tube 2 of the medical apparatus 101 which has a perforated barrier 13. The perforated barrier 13 extends along a vacuum port 22 which is connected to a longitudinal tissue receiving opening 23. The perforated barrier 13 has perforations for uniformly distributing the fluid supply and/or the applied suction. The perforated barrier 13 extends over the entire longitudinal tissue receiving opening 23 of the sizing tube 2 and is arranged to prevent tissue bulges from obstructing the vacuum port 22. The perforated barrier 13 is further arranged to form an abutment surface such that the tissue bulges can be punctured through the serosa. This enables to precisely control the tissue thickness of the received tissue bulges.

Figure 14C shows a perspective side view of a sizing tube 2 of the medical apparatus 101 having an expansion unit 11 which has a separation member 8. The expansion unit 11 is in an expanded configuration 112 for engaging the greater curvature of the stomach tissue (see Figs. 13D - 13G). The separation member 8 is formed by a meshed separation fabric which extends along a longitudinal receiving opening of the sizing tube 2 for separating a dorsal and ventral tissue bulge. A proximal end of the separation member is operatively connected to a proximal end of a nitinol wire of the expansion unit 11 and a distal end of the separation member 8 is connected to a distal end of the nitinol wire. The separation member 8 is deployable in unison with the expansion unit 11 being converted from a collapsed configuration to the expanded configuration 112. Simultaneous/synchronized deployment of the expansion unit 11 and the separation member 8 within the longitudinal tissue receiving opening allows for a more intuitive operation of the medical apparatus and a simpler design.

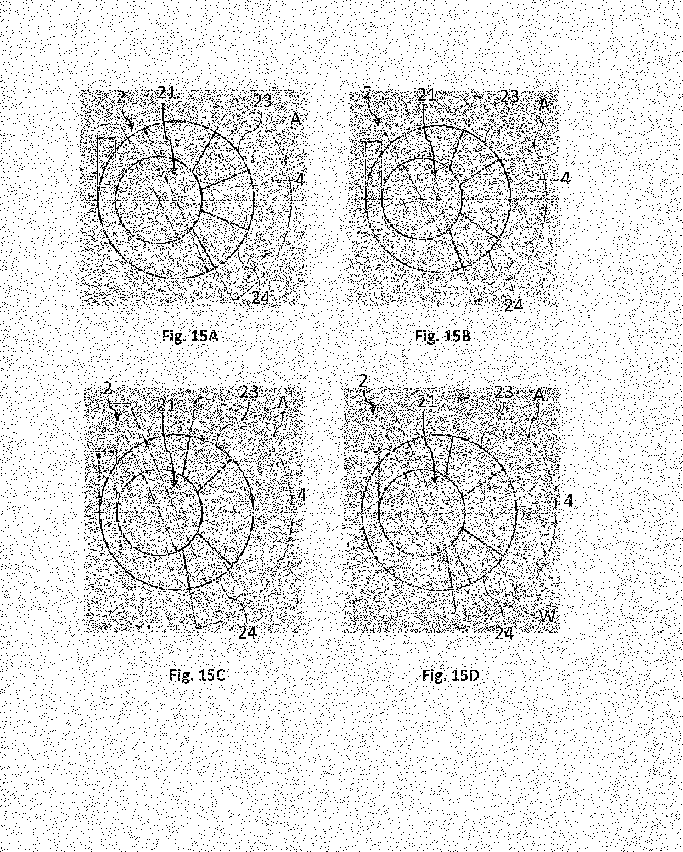

Figures 15A to ,15D show a cross-sectional view of different embodiments of the sizing tube 2 having circumferentially differently positioned and dimensioned longitudinal tissue receiving openings 23, 24. The longitudinal tissue receiving openings 23, 24 are separated by an elongated partitioner 4 and have a uniform cross-sectional profile along a longitudinal direction of the sizing tube 2.

The two longitudinal tissue receiving openings 23, 24 are ar-, ranged within a circumferential section of the sizing tube 2 having an angle A of 120° or less in Fig. 15A, 140° or less in Fig. 15B, and 160° or less in Figs. 15C and 15D. Arranging the tissue receiving openings 23, 24 within this angular range and aligned towards a greater curvature of the stomach allows to reliably engage the dorsal and ventral gastric wall to form and receive the tissue bulges and carry out an endosleeve surgery. Simultaneously, an opposing side of the sizing tube 2 facing away from the tissue receiving openings 23, 24 is aligned to face-the lesser curvature of the stomach.

Figure 15D further shows that an average width W of the longitudinal tissue receiving openings 23, 24 may be larger than shown in Figs. 15A - 15C. In a preferred embodiment, the width W may have a value between 4 mm - 6 mm.

Figures 15A - 15D further show that a cross-sectional geometric center of an inner lumen 21 is offset from a cross-sectional geometric center of the sizing tube 2. In this manner, the side of the sizing tube for engaging the lesser curvature of the stomach can have a thinner wall thickness, while the side for receiving the tissue bulges through the longitudinal tissue receiving openings 23, 24 can have a thicker wall thickness. This allows a miniaturization of the sizing tube 2 which facilitates insertion and at the same time allows for receiving desirably dimensioned tissue bulges due to a larger depth of the tissue receiving openings 23, 24. In addition, this may reduce the buckling of the elongated partitioner 4 and/or the sizing tube 2 when applying suction.

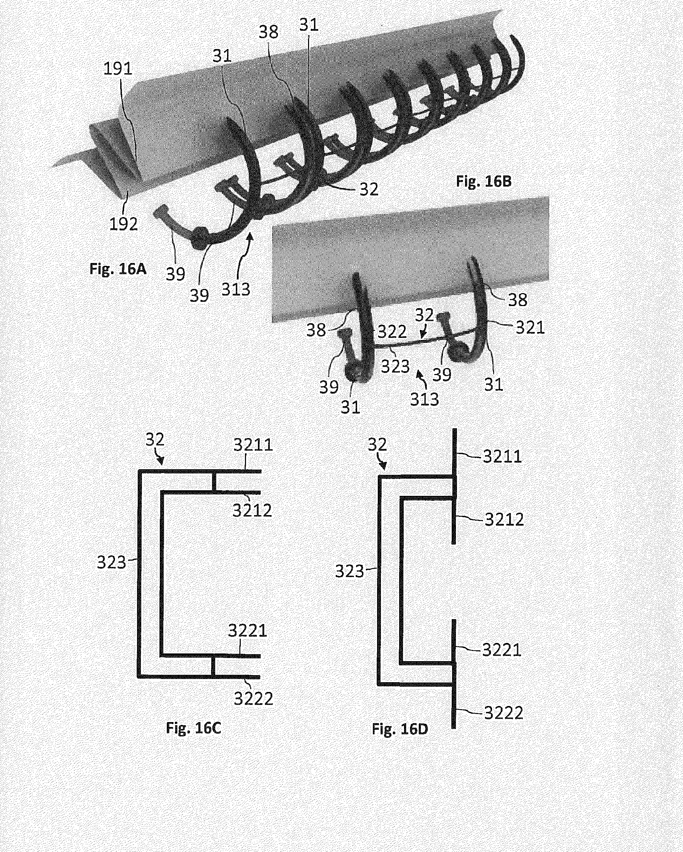

Figures 16A and 16B show a perspective view of a plurality of pairs 313 of puncturing elements 31 and a pair 313 of puncturing elements 31 with longitudinal slits 38. The slits face towards each other, respectively. The puncturing elements 31 are arranged in a puncturing connection unit which is not shown in Figs. 16A and 16B for better visibility of the puncturing elements 31. Figures 16A and 16B further show a fastener 32 that comprises a first and second fastening element 321, 322 which are connected by a connecting element 323 and which are arranged within inner lumen of each pair 313 of puncturing elements 31. The connecting elements 323 extend through the slits 38.. Fastener deployment members 39 which are formed by actuatable pistons are arranged within the inner lumen of the puncturing elements 31. The pairs 313 of puncturing elements and corresponding deployment members 39 are actuatable together such that the first and second fastening elements 321, 322 can be advanced through adjacently arranged dorsal and ventral tissue bulges 191, 192 to connect the tissue bulges 191, 192. In one embodiment, the plurality of pairs 313 of puncturing elements 31 and fastener deployment members 39 in Fig. 16A are actuatable simultaneously, in particular by an actuation unit operated by a control unit of the medical apparatus.

Deploying both the first and second fastening elements 321, 322 from the inner lumen of the pairs 313 of puncturing elements 31 allows for a more reliable positioning of the fasteners 32 and enables each fastener 32 to connect the tissue bulges 191, 192 at two separate puncture sites. In addition, a puncture resistance of advancing the fastener elements 321, 322 can be minimized since both fastener elements 321, 322 are deployed after successfully puncturing the tissue bulges 191, 192 such that surface interactions and/or frictional resistance for deployment is reduced.

Figures 16C and 16D show a schematic representation of an alternative embodiment of a fastener 32. The fastener 32 has a connecting element 323 which connects a first pair of fastening elements 3211, 3212 and a second pair of fastening elements 3221, 3222 which is formed by a u-shaped wire. This u-shape allows for reducing the strain on the gastric tissue when the fastener 32 of Figs. 16C and 16D is deployed through tissue bulges as previously described in Figs. 16A and 16B. The pairs of fastening elements 3211, 3212; 3221, 3222 are adapted to be deformed when being deployed through the tissue bulges. This may be achieved by a counter surface, e.g. formed by the puncturing connection unit, for receiving the pairs of fastening elements 3211, 3212; 3221, 3222 in such a manner that the fastening elements 3211, 3212; 3221, 3222 are bent laterally outwardly as shown in Fig. 16D. Thereby, detachment of the deployed fastener 32 through the tissue can be prevented since the laterally outwardly bent fastening elements 3211, 3212; 3221, 3222 form an anchoring surface against the tissue bulges.

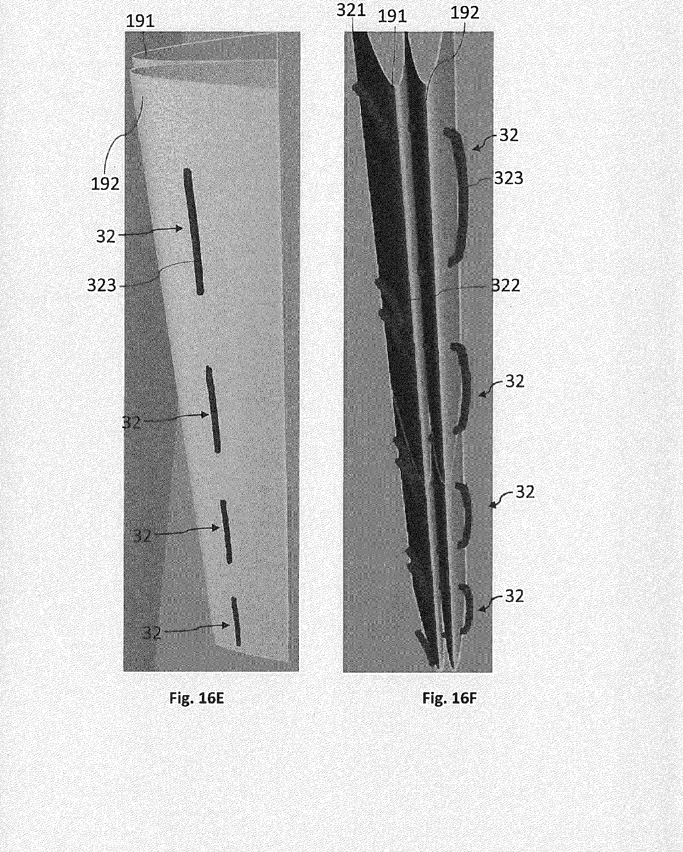

Figures 16E and 16F show perspective views of two tissue bulges 191, 192 which were connected by a plurality of fasteners 32 deployed via puncturing elements of Figs. 16A and 16B. The fastening elements 321, 322 of the fasteners 32 are arranged only on an exit side of the tissue bulges 191, 192. The connecting elements 323 of the fasteners 32 are U-shaped and extend through the tissue bulges 191, 192 longitudinally along an entry side of the tissue bulges 191, 192. This deployment of fasteners 32 enables each fastener 32 to securely connect the tissue bulges 191, 192 at two puncturing sites which are longitudinally spaced apart from each other along a longitudinal length of the tissue bulges 191, 192.

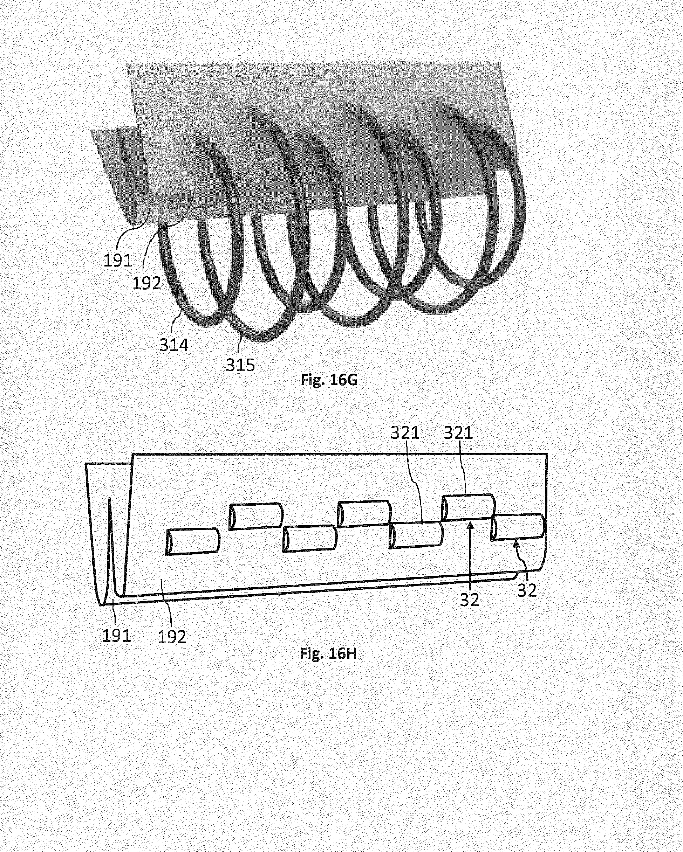

Figure 16G shows a perspective view of a plurality of puncturing elements 314, 315 with different diameters puncturing two tissue bulges 191, 192. The puncturing elements 314, 315 may be actuatable in a previously described manner in unison or separately from each other. The puncturing elements 314, 315 in Fig. 16G are arranged concentrically to a common axis corresponding to a longitudinal axis of a sizing tube to which the puncturing connection units of the puncturing elements 314, 315 are mounted (not shown in Fig. 16G). The first puncturing elements 314 having the smaller diameter are adapted for deploying a fastener 32 (see Fig. 16H) to connect the tissue bulges 191, 192 at a higher position with respect to the gastric wall where the tissue bulge dimension is smaller. The second puncturing elements 315 having the larger diameter are adapted for deploying the fastener 32 to connect the tissue bulges 191, 192 at a lower position with respect to the gastric wall where the tissue bulge dimension is larger. This allows placement of fasteners 32 in a zig-zag pattern.

Figure 16H shows a perspective view of two tissue bulges 191, 192 which were connected by a plurality of fasteners 32 deployed via the puncturing elements 314, 315 of Fig. 16G. The fasteners 32 are arranged in a zig-zag pattern which facilitates positioning a greater number of fasteners along the longitudinal extend of the tissue bulges 191, 192, ensuring a more robust linkage of the tissue bulges 191, 192. The fasteners 32 comprise fastening elements 321 which secure the fasteners 32 on either side of the tissue bulges 191, 192 in a previously described manner.

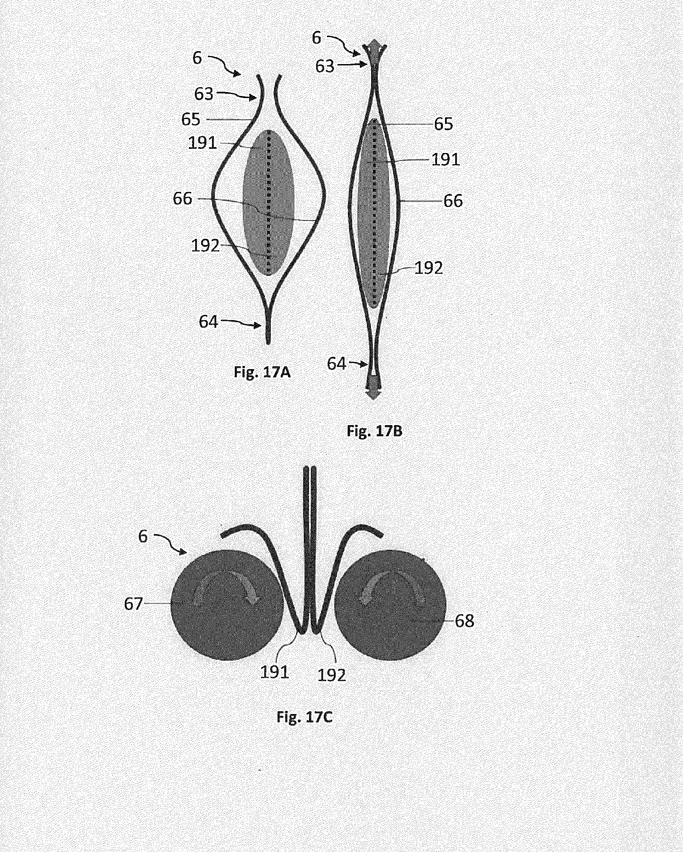

Figures 17A and 17B show a schematic view of an embodiment of a gripping means 6 for gripping tissue bulges 191, 192 via a radially collapsible structure. The tissue bulges 191, 192 in Figs. 17A and 17B are schematically separated by a dashed line. Alternatively, the medical apparatus may have two gripping means 6 of Figs. 17A and 17B which are configured for separately gripping one of the tissue bulges 191, 192 independently from each other, e.g. by being arranged within respective receiving openings of a sizing tube.

The collapsible structure of the gripping means 6 comprises two arcuate elements 65, 66 which are not connected at a first end 63 and joined at a second end 64. In a preferred embodiment, the arcuate elements may be connected to each other at the first and second ends. Figure 17A shows that the collapsible structure has a radially expanded configuration for receiving the tissue bulges 191, 192. Figure 17B shows that the collapsible structure can be moved to a radially collapsed configuration by moving at least one of the ends 63, 64 relative to the other of the ends 63, 64. This allows to apply a radially inwardly directed pressure in a uniform manner onto the tissue bulges 191, 192 by engaging the tissue bulges 191, 192 via the arcuate elements 65, 66 which are deformed to a lesser curvature. The collapsible structure may comprise more than two or three arcuate elements 165, 166 which are adapted for engaging the tissue bulges 191, 192, in particular similar to a Dormia device by Coloplast.

Figure 17C shows a schematic view of a different embodiment of a gripping means 6 for gripping the tissue bulges 191, 192 via two actuatable rollers 67, 68. The actuatable rollers 67, 68 of the gripping means 6 are formed by cylindrical rollers and are configured to be rotatable and in particular operable via a control unit of the medical apparatus. The actuatable rollers 67, 68 in Fig. 17C are arranged at least partially along a tissue receiving opening or tissue receiving openings. This allows that dimensions of the tissue bulges 191, 192 received within the tissue receiving opening(s) can be controlled in a more reliable manner. The dimensions of the tissue bulges 191, 192 can be adjusted via the actuatable rollers 67, 68 to optimize a position of a puncturing site via the puncturing element, e.g. through the serosa.

Figures 18A and 18B show a perspective side view of an embodiment of the sizing tube 2 of the medical apparatus. The sizing tube 2 has a puncturing connection unit 3 which is formed by a plurality of puncturing connection sub-units which each comprise four puncturing elements 31. The puncturing connection unit 3 extends over the entire longitudinal length of a tissue receiving opening 23 of the sizing tube 2. The puncturing connection unit 3 has an annular shape and is positioned circumferentially on a mantle or outer surface of the sizing.tube 2. Each of the puncturing connection sub-units are actuatable in unison with a corresponding gripping means 6. The gripping means 6 have a movable part 61. The movable part 61 is configured for gripping the tissue bulges and advancing the puncturing elements 31 formed by arcuate needles. A second non-movable part 62 of the gripping means 6 is adapted to interact with the first part 61 to grip the tissue bulges. The separate puncturing connection sub-units and corresponding gripping means 6 are configured to be actuatable independently from each other. This allows for sequential suturing of the tissue bulges along the tissue receiving opening(s) 23. This may provide facilitated application of suction to specific regions of the tissue receiving opening(s) 23 by partially closing the tissue opening(s) 23 by moving the sub-units sequentially to a.closed configuration.

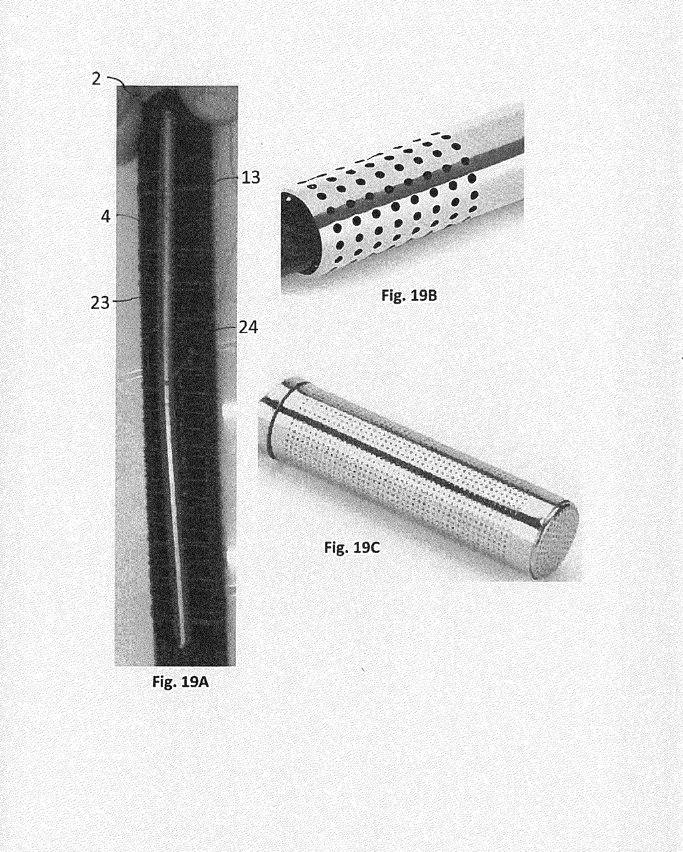

Figures 19A to 19C show a side view and two perspective views of different embodiments of a perforated barrier 13 of a sizing tube 2.

The perforated barrier 13 in Fig. 19A is formed by a wire which is wound around a sizing tube 2 below an elongated partitioner 4 such that tissue receiving openings 23, 24 of the sizing tube 2 are partially covered.

The perforated barrier 13 of the embodiments in Figs. 19B and 19C is formed by a perforated cylindrical segment which has as plurality of circular perforations which may have a diameter of 0.1 - 2 mm, preferably 0.5 - 1 mm.

The perforated barrier 13 in Figs. 19A to 19C allows a passage of fluid while preventing excess tissue of the tissue bulges to be sucked into a vacuum port of the sizing tube. In addition, the perforated barrier 13 is preferably oriented and positioned for controlling the dimensions of the tissue bulges which are formed within the receiving opening(s).

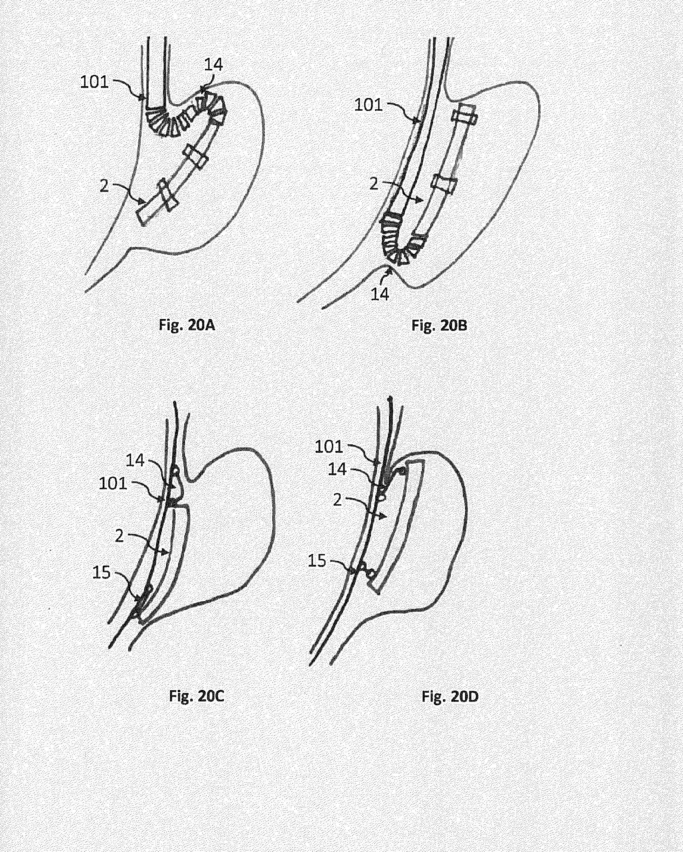

Figures 20A to 20D show a schematic representation of different embodiments of a medical apparatus 101 which comprises a sizing tube 2 that is movable by an articulated segment(s) 14, 15 of the medical apparatus 101.

The articulated segment 14 of the medical apparatus 101 in Figs. 20A and 20B is formed by a longitudinal section which has a proximal end connected to a delivery catheter and a distal end which is connected to a proximal end of the sizing tube 2. The articulated segment 14 may be actuatable via at least one deflection element, in particular at least.one actuatable tendon.

The at least one deflection element and routing of at least one deflection element may be configured for deflecting the articulated segments 14, 15 of the medical apparatus 101 along two distinct curvatures as shown in Fig. 20A or along a single curvature as shown in Fig. 20B.

A first articulated segment 14 of the medical apparatus 101 in Figs. 20C and 20D is connected to a proximal end of the sizing tube 2 and a second articulated segment 15 is connected to a distal end of the sizing tube 2. The articulated segments 14, 15 are formed by rigid links which are pivotable and allow positioning and/or orienting of the sizing tube 2 in a multi-bar linkage fashion known to a person skilled in the art.

The articulated segment(s) 14, 15 of Figs. 20A - 20D may comprise a manual actuation, a spring actuation, a pneumatic/hydraulic actuation, or an electric actuation such that the position/orientation of the sizing tube may be controlled, in particular via a control unit of the medical apparatus 101. This allows positioning the sizing tube 2 in a specific section of the stomach to adjust the dimensions of an endosleeve when performing an endosleeve stomach surgery by adjusting an angular orientation and position of the sizing tube 2.

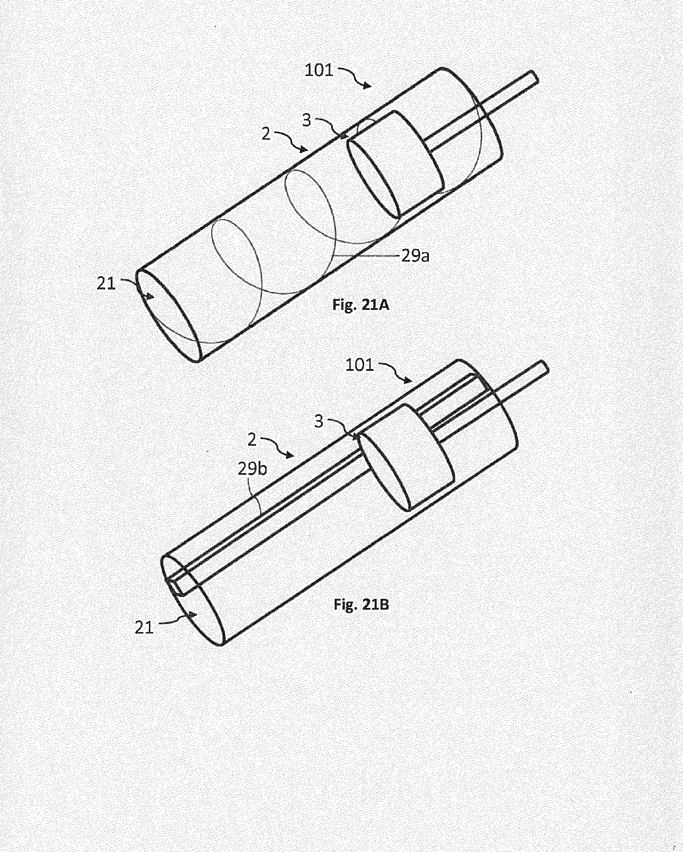

Figures 21A and 21B show a semi-opaque perspective view of two embodiments of the medical apparatus 101 having a sizing tube 2 with a helical guiding feature 29a and a longitudinal guiding feature 29b, respectively. A puncturing connection unit 3 of the medical apparatus 101 has an engagement section to engage the guiding feature 29a, 29b.

The guiding feature 29a in Fig. 21A has a helical shape which extends in a longitudinal direction within an inner lumen 21 of the sizing tube. The guiding feature 29a is configured for guiding the puncturing connection unit 3 along a helical path within the inner lumen 21. The puncturing connection unit 3 is driven along the guiding feature 29a by rotation and translation of a push rod which is connected to a proximal end of the puncturing connection unit 3.

The guiding feature 29b in Fig. 21B has a longitudinal guide rail which extends parallel to a longitudinal direction of the sizing tube 2. This allows the puncturing connection unit 3 to be translationally movable along a linear path in the inner lumen 21 along the sizing tube 2 by being driven by translationally advancing a push rod connected to a proximal end of the puncturing connection unit 3.

The puncturing connection unit 3 may comprise an electronic control unit for operating a puncturing element and deploy sutures/fastener which are pre-loaded within the puncturing connection unit 3, in particular in a replenishing reservoir/magazine.

The guiding feature 29a, 29b may extend longitudinally along a tissue receiving opening(s) of the sizing tube 2 such that the puncturing connection unit 3 may be guided along the tissue receiving opening(s) to connect the dorsal and ventral tissue bulges within the tissue receiving opening(s). In particular, the helical shape of a guiding feature 29a may allow for deploying sutures/fasteners at specific predetermined positions corresponding to the windings of the helical shape. The helical guiding feature 29a may be partially discontinuous, in particular in the region of the tissue receiving opening(s).

## Claims

1. A medical apparatus (101) for carrying out an endoscopic gastric remodelling procedure comprising
- a sizing tube (2) which has
∘ an inner lumen (21),
∘ at least one vacuum port (22) for applying suction to stomach tissue (19) and forming at least two tissue bulges (191, 192) of the stomach tissue (19),
∘ at least one tissue receiving opening (23, 24) extending in a longitudinal direction (L) of the sizing tube (2) for receiving the tissue bulges (191, 192) based on the applied suction,
- at least one puncturing connection unit (3), **characterized in that** the puncturing connection unit (3) is adapted for connecting the at least two tissue bulges (191, 192) with each other by puncturing the tissue bulges (191, 192), and
- at least one expansion unit (11) for spreading the stomach, the expansion unit (11) having a collapsed configuration (111) and an expanded configuration (112), wherein at least one of the expansion unit (11) in the expanded configuration is configured for engaging the greater curvature (196) of the stomach and for displacing the greater curvature (196) away from the sizing tube (2).

2. The medical apparatus (101) according to claim 1, wherein the expansion unit (11) comprises at least one of
- - an inflatable structure which has a collapsed configuration and an expanded configuration, wherein the inflatable structure is adapted for displacing the greater curvature (196) away from the sizing tube (2) in the expanded configuration, and
- an elongated element, in particular a hyperelastic elongated element, which.has a collapsed configuration and an expanded configuration, wherein the elongated element in the expanded configuration is adapted for displacing the greater curvature away from the sizing tube (2), in particular by the elongated element interfacing at least partially tangentially with the greater curvature, and wherein the elongated element preferably has an arcuate shape with a smaller radius in the expanded configuration than in the collapsed configuration.

3. The medical apparatus (101) according one of the preceding claims, wherein the apparatus (101) has at least two tissue receiving openings (23, 24) which are connected to the inner lumen (21) of the sizing tube (2) and are at least partially separated by an elongated partitioner (4) of the sizing tube (2), wherein the first tissue receiving opening (23, 24) is adapted for receiving the first tissue bulge (191) and the second tissue receiving opening (23, 24) is adapted for receiving the second tissue bulge (192) when the suction is applied.

4. The medical apparatus (101) according to claim 3, wherein the elongated partitioner (4) of the sizing tube (2) has at least one of the following:
- one free end (41) which is not connected to the sizing tube (2) and one joined end (42) which is connected to the sizing tube (2),
- two joined ends (42, 43) connected to the sizing tube (2) with at least one of the joined ends (42, 43) being separable from the sizing tube (2),
- a non-flexible state, in particular a non-flexible state having a bent shape, which can be converted into a more flexible state by a conversion element, in particular by retraction of the conversion element from the elongated partitioner (4), wherein
in the flexible state, a plurality of separated longitudinal subsections (44) of the elongated partitioner (4) are preferably freely movably connected to each other by at least one flexible connecting element (45), in particular a cable,
- a self-dissolving, soluble, and/or bioresorbable material.

5. The medical apparatus (101) according to one of the preceding claims, wherein the inner lumen (21) of the sizing tube (2) has a first terminal opening (25) and a second terminal opening (26) on an opposing side from the first terminal opening (25), such that the sizing tube (2) may be advanced over a medical instrument (201), in particular a guidewire, a balloon catheter, and/or an endoscope (202), wherein the medical apparatus (101) preferably comprises the medical instrument (201) .

6. The medical apparatus (101) according to one of the preceding claims, wherein the medical apparatus (101) has at least one sealing unit (5) for sealing an outlet (193) from the stomach and/or an inlet (194) into the stomach, wherein the sealing unit (5) in particular comprises a first sealing element (51) and a second sealing element (52), preferably formed by a balloon, and the first sealing element (51) is adapted for sealing the outlet (193) and the second sealing element (52) is adapted for sealing the inlet (194) .

7. The medical apparatus (101) according to one of the preceding claims, wherein the sizing tube (2) is at least partially radially expandable, in particular inflatable, preferably along an entire longitudinal length of the sizing tube (2), and the sizing tube (2) is in particular sized and shaped to be radially expandable from a first cross-sectional dimension of the sizing tube (2) having a value between 1 mm to 20 mm, in particular 2 mm to 16 mm, preferably 4 mm to 12 mm,
to a second cross-sectional dimension having a value between 10 mm - 35 mm, in particular 15 mm - 25 mm, preferably 18 mm - 25 mm.

8. The medical apparatus (101) according to one of the preceding claims, wherein a volume of the sizing tube (2), or at least the section of the sizing tube (2) adapted for insertion into the stomach, is between 50 ml - 350 ml, in particular 75 ml - 250 ml, preferably 100 ml - 200 ml.

9. The medical apparatus (101) according to one of the preceding claims, wherein the puncturing connection unit (3) is arranged or arrangeable:
- partially or completely within an inner lumen (21).of the sizing tube (2), in particular partially within the at least one tissue receiving opening (23, 24),
- around a medical instrument (202) within the sizing tube (2),
- around the sizing tube (2) and
- around an elongated partitioner (4) of the sizing tube (2) formed between two openings (23, 24), in particular formed between two tissue receiving openings (23, 24), of the sizing tube (2).

10. The medical apparatus (101) according to one of the preceding claims, wherein the puncturing connection unit (3) is longitudinally movably arranged or arrangeable with respect to the sizing tube (2), in particular slidable along a guiding feature (27) of the sizing tube (2).

11. The medical apparatus (101) according to one of the preceding claims, wherein the sizing tube (2) has a bent shape (28) or is deflectable to a bent shape (28), wherein the bent shape (28) preferably has an angle between 10° - 110°, in particular between 20° - 100°, over an entire or partial longitudinal extension of the sizing tube (2), wherein the longitudinal extension is in particular shaped or deflectable corresponding to the gastric course along the lesser curvature (195) of the stomach.

12. The medical apparatus (101) according to one of the preceding claims, wherein the puncturing connection unit (3) has at least one puncturing element (31), in particular a curved puncturing element (31), which is functionally connected or connectable to a connecting structure, in particular to a fastener (32) or a suture (33), wherein
the puncturing connection unit (3) is adapted for deploying the connecting structure through the two tissue bulges for connecting the tissue bulges (191, 192) by advancing the puncturing element (31) with the.connecting structure through the tissue bulges (191, 192) and preferably retracting the puncturing element (31).

13. The medical apparatus (101) according to claim 12, wherein the medical apparatus (101) comprises at least one fastener (32), wherein the fastener (32) is I-shaped or U-shaped and has a first fastening element (321) and a second fastening element (322) which are connected by a connecting element (323), wherein at least one fastening element (321, 322) is preferably arranged or arrangeable at least partially circumferentially around the puncturing element (31) or within the puncturing element (31).

14. The medical apparatus (101) according to one of the preceding claims, wherein the puncturing connection unit (3) has a hydraulic, pneumatic, magnetic, or mechanic actuation unit for actuating the puncturing element (31), in particular for advancing and retracting the puncturing element (31) through the tissue bulges (191, 192).

15. The medical apparatus (101) according to one of the preceding claims, wherein the medical apparatus (101) comprises a gripping means (6), in particular formed by the puncturing connection unit (3), which is configured to adjoin the two tissue bulges (191, 192) together, prior to connecting the tissue bulges (191, 192), in particular by engaging the two tissue bulges (191, 192) from opposing sides, so that they can be connected by the puncturing connection unit (3).

16. The medical apparatus (101) according to one of the preceding claims, wherein the medical apparatus (101) comprises an implantable layer (7) which is pre-loaded or loadable within the inner lumen (21) of the sizing tube (2) which is attachable to the tissue bulges (191, 192) when connecting the two tissue bulges via the puncturing connection unit (3), wherein
the implantable layer (7) is preferably adapted for confining the two tissue bulges (191, 192) within a partial cross-sectional volume of the inner lumen (21) when suction is applied by the at least one vacuum port (22), in particular by the implantable layer (7) being connected to two lateral opposing sections of the sizing tube (2).

17. The medical apparatus (101) according to claim 16, wherein the implantable layer (7) comprises or consists of at least one of
- a haemostatic component for reducing bleeding,
- a drug delivery layer which is adapted for enhancing haemostasis and/or reducing scarring, and
- a waterproof layer for rendering a connection region of the two tissue bulges (191, 192) less permeable to fluids, wherein the waterproof layer is formed by a synthetic and/or a biological material.

18. The medical apparatus (101) according to one of the preceding claims, wherein the medical apparatus (101) has a separation member (8), in particular having a flexible separation fabric, which is deployable laterally outwardly with respect to the sizing tube (2) extending through one tissue receiving opening (23, 24) of the sizing tube (2) for separating the two tissue bulges (191, 192) from each other along the longitudinal direction (L) when applying suction.

19. The medical apparatus (101) according to claim 18, wherein the expansion unit (11) comprises the separation member (8), and wherein the expansion unit (11) is preferably adapted for collapsing and deploying the separation member (8) when being converted between the collapsed and expanded configuration.

20. The medical apparatus (101) according to one of the claims 18 or 19, wherein the medical apparatus (101) has an elongated deployment member (9) which is connected or connectable to the separation member (8) and adapted to
- reversibly deploy the separation member (8) by pivoting the elongated deployment member (9) laterally outwardly, or
- reversibly deploy the separation member (8) by longitudinally advancing the elongated deployment member (9) such that a section of the elongated deployment member (9) which is connected to the separation member (8) moves laterally outwardly with respect to the sizing tube (2).

21. The medical apparatus (101) according to one of the preceding claims, wherein the medical apparatus (101) comprises a robotic system for carrying out the endoscopic gastric remodelling procedure in a partially autonomous manner, in particular in a fully autonomous manner.

22. A method for carrying out an endoscopic gastric remodelling procedure using a medical apparatus (101), in particular a medical apparatus (101) according to one of the preceding claims, the method comprising the steps of:
- Inserting a sizing tube (2) of the medical apparatus (101) perorally across the oesophagus into the stomach of a patient,
- Optionally sealing an outlet () and/or an inlet () of the stomach using a sealing unit (5) of the medical apparatus (101),
- Optionally laterally outwardly deploying a separation member through a tissue receiving opening of the sizing tube (2) by pivoting or longitudinally advancing an elongated deployment member (),
- Optionally converting an expansion unit () of the medical apparatus (101) for spreading the stomach from a collapsed configuration () to an expanded configuration ().
- Applying suction via at least one vacuum port (22) of the sizing tube (2) to form two tissue bulges (191, 192) of the stomach tissue (19),
- Optionally moving a puncturing connection unit (3) of the medical apparatus (101) in a longitudinal direction (L) with respect to the sizing tube (2), and
- connecting the two tissue bulges (191, 192) with each other by puncturing the tissue bulges.
